# FASCICULE DE BREVET EUROPEEN

(11) **EP 3 188 743 B1**
(45) Date de publication et mention de la délivrance du brevet: **18.11.2020**
(21) Numéro de dépôt: 15778354.9
(22) Date de dépôt: 04.09.2015
(51) Int. Cl.: A61K 36/8998, A61K 36/899, A61P 1/16

(54) **TRAITEMENT DE LA TOXICITÉ HÉPATIQUE ET CÉRÉBRALE INDUITE PAR AU MOINS UN POLLUANT**
BEHANDLUNG VON HEPATISCHE UND ZEREBRALE TOXIZITÄT VERUSACHT DURCH MINDENSTENS EINEN SCHADSTOFF
TREATMENT OF HEPATIC AND CEREBRAL TOXICITY CAUSED BY AT LEAST ONE POLLUTANT

(30) Priorité: 05.09.2014 FR 1458341
(43) Date de publication de la demande: 12.07.2017
(73) Titulaire: Université Grenoble Alpes, 38400 Saint Martin d'Hères (FR); Centre National de la Recherche Scientifique, 75794 Paris Cedex 16 (FR)
(72) Inventeur: DEMEILLIERS-DARBLADE, Christine Arlette, F-38920 Crolles (FR); OUNNAS, Fayçal, 57400 Sarrebourg (FR); DE LORGERIL, Michel, F-38240 Meylan (FR); SALEN, Patricia, F-38240 Meylan (FR)
(74) Mandataire: Cabinet Chaillot
(86) Numéro de dépôt international: PCT/FR2015/052352
(87) Numéro de publication internationale: WO 2016/034828

(56) Documents cités:
- WO-A1-2007/048921
- WO-A1-2010/059624
- JP-A- 2000 245 392
- JP-A- 2003 055 252
- US-A1- 2004 258 776
- US-A1- 2005 271 600
- VERMEYLEN ET AL: "Effects of dietary wheat bran on absorption and accumulation of PCBs in rats", CHEMOSPHERE, vol. 71, no. 2, 30 octobre 2007 (2007-10-30), pages 277-283, XP022489613, PERGAMON PRESS, OXFORD, GB ISSN: 0045-6535, DOI: 10.1016/J.CHEMOSPHERE.2007.09.026
- IIDA T ET AL: "Clinical trial of a combination of rice bran fiber and cholestyramine for promotion of fecal excretion of retained polychlorinated dibenzofuran and polychlorinated biphenyl in Yu-Chen patients", FUKUOKA ACTA MEDICA, vol. 86, no. 5, 1995, pages 94-101, XP009182564, JP ISSN: 0016-254X
- SERA N ET AL: "Binding effect of polychlorinated compounds and environmental carcinogens on rice bran fiber", JOURNAL OF NUTRITIONAL BIOCHEMISTRY, vol. 16, no. 1, 1 janvier 2005 (2005-01-01), pages 50-58, XP027618736, BUTTERWORTH PUBLISHERS, STONEHAM, GB ISSN: 0955-2863 [extrait le 2005-01-01]
- Anonymous: "Horizon Millings GrainWise Wheat Aleurone helps food manufacturers create all-natural, nutrient-rich products", Cargill , 28 janvier 2008 (2008-01-28), XP002735946, Extrait de l'Internet: URL:http://www.cargill.com/news/releases/2 008/NA3007660.jsp [extrait le 2015-02-13]
- OUNNAS ET AL.: "P015: Exposition « réaliste » à un mélange de polychlorobiphényles (PCB) inclus dans une matrice alimentaire : toxicité et prévention", NUTRITION CLINIQUE ET METABOLISME, vol. 28, 23 décembre 2014 (2014-12-23), XP002735947, DOI: 10.1016/S0985-0562(14)70658-2
- Yasuhiro Kimura ET AL: "Nutrient Interactions and Toxicity Some Dietary Fibers Increase Elimination of Orally Administered Polychlorinated Biphenyls but Not That of Retinol in Mice 1", , 1 janvier 2003 (2003-01-01), XP055224932, Extrait de l'Internet: URL:http://jn.nutrition.org/content/134/1/ 135.full.pdf#page=1&view=FitH [extrait le 2015-11-02]
- "ATSDR Case Studies in Environmental Medicine Polychlorinated Biphenyls (PCBs) Toxicity", , 14 mai 2014 (2014-05-14), XP55224733, Extrait de l'Internet: URL:http://www.atsdr.cdc.gov/csem/pcb/docs /pcb.pdf [extrait le 2015-10-30]
- Andrea M Zander: "Part I: Heavy Metal Poisoning Signs & Symptoms 1", , 1 janvier 2012 (2012-01-01), XP055224935, Extrait de l'Internet: URL:http://www.roblongo.com/pdfs/Heavy Metal Toxicity.pdf [extrait le 2015-11-02]

## Description

L'invention concerne un procédé de traitement préventif ou curatif des altérations induites par les polluants organiques persistants (POP) notamment de type polychlorobiphényles (PCBs) et/ou dioxines et/ou tout polluant ayant des effets similaires tels que les pesticides et/ou les métaux lourds.

Les POP sont des molécules toxiques pour la santé et l'environnement, d'origines naturelles et/ou issues des activités humaines, qui se caractérisent par leur résistance aux dégradations biologiques naturelles et de ce fait persistent dans l'environnement entrainant notamment leur accumulation dans les tissus vivants. En raison de ces propriétés de persistance et de bioaccumulation, les POP ont la capacité de se déposer loin de leurs lieux d'émission, et ainsi d'accroitre l'étendue de leur présence dans l'environnement.

Parmi les POP, on distingue notamment les PCBs qui correspondent à des composés organiques chlorés qui contaminent régulièrement l'alimentation humaine et les cours d'eau, les dioxines, issues des combustions naturelle ou dues à des procédés industriels, et d'autres polluants ayant des effets similaires tels que les pesticides qui sont des substances répandues sur les cultures pour lutter contre des organismes considérés comme nuisibles, comprenant les insecticides, les fongicides, les herbicides, les parasiticides.

Les PCBs désignent une famille de composés de synthèses organochlorés dérivés du biphényle, de haut poids moléculaire et de formule chimique C₁₀H₁₀-nClₙ.

Les PCBs s'organisent en deux groupes, l'un contenant des PCB dioxin like (PCB-DL) et l'autre des PCB non dioxin-like (PCB-NDL), qui sont identifiés par l'Union Européenne (EFSA - European food safety authority, 2005. Opinion of the scientific panel on contaminants in the food chain on a request from the commission related to the presence of non dioxin-like polychlorinated biphenyls (PCB) in feed and food, EFSA J. 284, 1-137) (EFSA, 2010. Scientific Report of EFSA. Results of the monitoring of non dioxin-like PCBs in food and feed. EFSA J. 8 (7), 1701).

Produits industriellement depuis 1930 sous le nom de pyralènes, les mélanges de PCBs ont fait l'objet de multiples utilisations comme produits isolants pour les transformateurs électriques, additifs dans les peintures, les encres et les apprêts destinés aux revêtements muraux. Cependant, après avoir découvert les impacts de leur large dissémination dans les lacs et les océans, ils ont progressivement été interdits à partir des années 80.

Les émissions de PCBs dans l'environnement proviennent également de procédés industriels divers faisant intervenir la combustion incomplète de dérivés aromatiques chlorés ou impliquant la synthèse de dérivés chlorés (incinération de déchets, fonderie, métallurgie, sidérurgie, brûlage de câbles, fabrication d'herbicides et de pesticides, etc.).

Ces PCBs ont une durée de vie très longue, et du fait de leur lipophylie, ils s'accumulent dans les réserves lipidiques des animaux, ce qui entraine leur transfert tout au long de la chaine alimentaire ; leur concentration dans les organismes augmentant ainsi avec leur niveau trophique (bioamplification).

A l'exception de l'exposition professionnelle ou accidentelle (Masuda et al., 1998, Concentrations of PCBs, PCDFs and PCDDs in the blood of Uusho patients and their toxic équivalent contribution. Chemospere. 37, 1773-1780), les produits animaux contribuent largement à l'exposition humaine aux PCBs. Les poissons gras, la viande et les produits laitiers représentent les sources majeures de cette exposition (AFSSA - Agence Française de la Sécurité Sanitaire des Aliments, 2006, Etablissement de teneurs maximales pertinentes en polychlorobiphényles qui ne sont pas de type dioxine (PCB « non dioxin-like », PCB, NDL) dans divers aliments. Afssa-Saisine n°2006-SA-0305, 1-28). Liem et al. (2000, Exposure of populations to dioxins and related compounds. Food Addit. Contam. 17, 241-259) ont estimé à 90% la contribution de l'alimentation à l'exposition totale de l'homme aux PCBs et aux PCDD/Fs, dioxines qui regroupent les polychlorobenzodioxines (PCDD) et les polychlorodibenzofuranes (PCDF).

Selon l'Agence Française de Sécurité Sanitaire des Aliments (AFSSA), l'exposition moyenne aux PCB-DL par l'alimentation, en tenant compte des dioxines et des furanes qui appartiennent à cette famille de composés, est estimée à 1,8 pg quantité équivalente toxique (TEQ)/kg PC/jour chez les adultes et à 2,8 pg TEQ/kg PC/jour chez les enfants de 3 à 14 ans, alors que la Dose Journalière Admissible (DJA) est fixée à 2,33 pg TEQ/kg PC/jour. Etant donné que les valeurs ci-dessus correspondent à des moyennes, l'AFSSA estime ainsi que la DJA serait dépassée pour 20 à 28 % de la population (AFSSA, 2006. Etablissement de teneurs maximales pertinentes en polychlorobiphenyles qui ne sont pas de type dioxine (PCB « non dioxin-like », PCB-NDL) dans divers aliments. Maisons-Alfort, le 23 octobre 2007. Saisine n° 2006-SA 0305) (Données CIRE-Rhône-Alpes, Janvier 2010).
Pour l'ensemble des composés de la famille des PCBs, la DJA est fixée à 20 ng/kg PC/jour. Les PCBs indicateurs comprennent un PCB DL à savoir le PCB 118 et les PCBs NDL suivants : PCB 28, PCB 52, PCB 101, PCB 138, PCB 153 et PCB 180. Ils représentent à eux seuls environ 50% de l'ensemble des PCBs, leur exposition moyenne est estimée à 8,8 ng/kg PC/jour chez les adultes et à 14,9 ng/kg PC/jour chez les enfants de 3 à 14 ans, alors que leur DJA est fixée à 10 ng/kg PC/jour. Ainsi, un dépassement de la DJA concernerait 58 % des enfants et 20 % des adultes.

Une fois dans l'organisme, ces composés peuvent entrainer des problèmes sur le long terme. En effet, l'exposition alimentaire et la bioaccumulation des PCBs pourraient (1) être impliqués dans l'augmentation de l'incidence des cancers du foie et du poumon, (2) avoir un impact sur les capacités d'apprentissage et de mémorisation, (3) avoir plusieurs conséquences sur le plan hormonal (désordres menstruels et problèmes de reproduction chez la femme, hypothyroïdie) et (4) augmenter le risque de diabète de type 2 (Crinnion WJ., 2011, Pollutants with immunological, neurological, and endocrinological consequences. Altern. Med. Rev ; 16 (1) :5-13). De plus, les données de la littérature actuelle suggèrent que l'exposition *in utero* pourrait induire un retard de développement sur le plan neurologique, augmenter l'incidence d'infections de types ORL et entrainer une déficience sur le plan immunitaire (Crinnion WJ., 2011, Pollutants with immunological, neurological, and endocrinological consequences. Altern. Med. Rev ; 16 (1) :5-13).

Parmi les mécanismes moléculaires impliqués, il a été démontré que les PCBs administrés à des doses élevées allant du µg/kg à plusieurs mg/kg, entrainent une augmentation de la mort cellulaire ainsi qu'une augmentation du stress oxydant (Jonsson et al., 1981, Effects of prolonged exposure to dietary DDT and PCB on rat liver morphology. ARCH. Environm. Contam. Toxicol. 10, 171-183) (Shertzer et al., 2006, TCDD decreases ATP levels and increases reactive oxygen production through changes in mitochondrial F0F1-ATP synthase and ubiquinone. Environ. Health. 217(3), 363-374). De plus, il a été décrit que les PCBs induisaient des altérations mitochondriales (Forgacs et al., 2010, Effects of TCDD on the expression of nuclear encoded mitochondrial genes. Toxico. Appl. Pahrm., 246(1-2), 58-65) (Tappenden et al., 2011, The aryl hydrocarbon receptor interacts with ATP5α1, a subunit of the ATP synthase complex, and modulates mitochondrial function. Toxico. Appl. Pharm. 254(3), 299-310). D'autres études menées *in vitro* ont permis de mettre en évidence un effet toxique direct sur des mitochondries hépatiques isolées. En effet, à titre d'exemple, il a été montré que le 4-Hydroxy-3,5,3',4'-Tetrachlorobiphényl induit un relargage du calcium, la génération d'espèces réactives de l'oxygène, une dépolarisation, un gonflement mitochondrial et un relargage du cytochrome c (Fujita et al.,2006, 4-Hydroxy-3,5,3',4'-Tetrachlorobiphenyl induced membrane permeability transition in isolated rat liver mitochondria. J. Clin. Biochem. Nutr., 38, 167-175).

Les risques d'exposition aux POP de type PCBs sont devenus aujourd'hui un enjeu de santé publique du fait de leur récurrence et de leur dispersion globale dans l'environnement.

De plus, l'élimination complète de ces contaminants persistants dans l'environnement est extrêmement difficile, très couteuse et de plus, aboutit généralement à la génération d'autres types de polluants.

Au vu de l'état de l'art antérieur, il faut noter que les études précédemment citées sur les PCBs (*in vivo* et *in vitro)* ont été réalisées le plus souvent sur la base de l'utilisation d'une molécule unique, par exemple la TCDD (2,3,7,8-tétrachlorodibenzo-p-dioxine) ou le PCB 126 (3,3',4,4',5-pentachlorobiphényle) (les plus toxiques) ou sur la base de mélanges commerciaux spécifiques tel que l'Aroclor® 1254, et à des doses élevées allant de quelques µg à plusieurs mg selon les études. Cependant, ces expositions ne reflètent pas la réalité d'un contact quotidien, c'est-à-dire du contact répété avec de faibles quantités d'un mélange complexe de PCBs par le biais de l'alimentation à laquelle un individu est soumis quotidiennement.

Les pesticides sont des composés chimiques dotés de propriétés toxicologiques, utilisés par les agriculteurs pour lutter contre les animaux (insectes, rongeurs) ou les plantes (champignons, mauvaises herbes) jugés nuisibles aux plantations. Leur toxicité, liée à leur structure moléculaire, ne se limite pas en effet aux seules espèces que l'on souhaite éliminer, ils sont notamment toxiques pour l'homme et des études attestent le lien entre exposition aux pesticides et survenue de certaines pathologies. Les principaux pesticides utilisés actuellement appartiennent à quelques grandes familles chimiques : les organochlorés, les organophosphorés, les pyréthroïdes, les carbamates, les phytosanitaires regroupant un très grand nombre de produits de la famille des triazines.

Les métaux lourds sont présents naturellement dans l'environnement et sont utilisés industriellement. La plupart se retrouvent donc dans l'alimentation. Certains sont essentiels au bon fonctionnement de l'organisme humain tandis que d'autres n'ont aucune fonction biologique. Mais en excès, tous peuvent présenter des risques pour la santé.

L'arrêté du 2 février 1998, relatif aux contaminations et aux émissions des installations classées pour la protection de l'environnement, fixe notamment les émissions de toute nature que doivent respecter ces installations. L'arrêté sélectionne un certain nombre de métaux sujets à des contraintes environnementales. Douze métaux sont concernés : Aluminium, Arsenic, Cadmium, Chrome, Nickel, Cuivre, Etain, Fer, Manganèse, Mercure, Plomb, Zinc.

En particulier l'Arsenic, le Plomb, le Mercure et le Cadmium sont dangereux :
- en cas d'exposition à une dose importante (toxicité aigue)
- en cas d'exposition longue à des doses plus faibles (accumulation dans les tissus et toxicité chronique).

L'exposition chronique, peut causer maladies et vieillissement accéléré.

L'aleurone est généralement présente sous forme de granules, les grains d'aleurone, dans l'assise externe de l'albumen des céréales, appelée couche à aleurone.

Cette couche histologique assure pour la plante à la fois un rôle nourricier via le stockage de métabolites et la synthèse d'enzymes d'hydrolyse des réserves, et un rôle de protection. C'est un tissu complexe qui renferme des concentrations importantes de molécules bioactives d'intérêt nutritionnel important (polyphénols, vitamines, minéraux...). Elle représente une source potentielle de micronutriments qui sont aujourd'hui peu valorisés, puisque en meunerie la couche à aleurone est éliminée lors de la mouture avec le péricarpe et le tégument du grain, dans la fraction du son.

Dans l'art antérieur, diverses utilisations de l'aleurone ont déjà été décrites.

La demande WO 2010/059 624 concerne l'utilisation de l'aleurone pour le traitement, le maintien, ou l'amélioration de la qualité des matières fécales et de la santé gastro-intestinale. En effet, l'aleurone agit sur la sécrétion de la bile par le foie, sur la dégradation des amines, l'urée, la composition bactérienne dans les intestins.

Le brevet US 2004/ 0 258 776 décrit une composition comprenant de l'aleurone, notamment extrait du son de blé, pour son administration dans l'alimentation humaine et animale en raison de ses propriétés physiologiques favorables, car l'aleurone de blé contient sous forme concentrée des vitamines, des substances minérales, des fibres, des protéines (albumine) et d'autres substances bioréactives telles que les phénols et les flavonoïdes.

Le brevet US 5 728 384 concerne l'utilisation d'une composition pharmaceutique à base d'aleurone obtenu à partir du riz, comme agent anti ulcéreux.

Le brevet européen EP 0 906 760 a pour objet l'utilisation d'une composition comprenant une couche à aleurone pour la préparation d'un médicament pour le traitement de la colite ulcéreuse, et plus particulièrement l'utilisation d'une composition comprenant des protéines et des fibres alimentaires insolubles, dont l'aleurone, qui sont isolés à partir de la graine germée d'une plante de la famille des herbacées, pour traiter les colites ulcéreuses et les effets secondaires des disfonctionnements intestinaux.

Le brevet européen EP 2 367 445 divulgue une amélioration de la santé intestinale via l'utilisation d'aleurone, et décrit notamment que l'utilisation des substances bioactives de la couche à aleurone permet de protéger l'organisme contre certaines formes de cancer ou de contribuer à une bonne santé métabolique.

Enfin la demande KR 2003 071 711 décrit une composition comprenant une couche à aleurone et des germes de riz, ladite composition étant susceptible d'améliorer la fonction hépatique.
L'un des aspects de la présente invention est de développer un traitement préventif ou curatif des altérations induites par les polluants organiques persistants (POP) en raison de leur ingestion chronique.
L'un des aspects de la présente invention est de développer un traitement préventif ou curatif des altérations induites par les pesticides en raison de leur ingestion chronique.
L'un des aspects de la présente invention est de développer un traitement préventif ou curatif des altérations induites par les métaux lourds en raison de leur ingestion chronique.

Un autre aspect de la présente invention est de développer une nouvelle utilisation des déchets de céréales et de ce fait de permettre une valorisation des déchets de l'industrie céréalière.

La présente invention vise à fournir une composition comprenant tout ou une partie de la couche à aleurone d'une céréale pour son utilisation dans la prévention ou le traitement des altérations induites par au moins un polluant, notamment un polluant organique persistant.

La présente invention a donc pour objetune composition comprenant tout ou partie de la couche d'aleurone d'une céréale pour son utilisation dans la prévention ou le traitement de la toxicité hépatique et cérébrale induite par au moins un polluant, notamment contenu dans un aliment,
ledit polluant étant un PCB ou un mélange de PCBs et/ou un pesticide choisi parmi les organochlorés, les organophosphorés, les pyréthroïdes, les carbamates et les triazines,
la quantité d'aleurone administrée étant comprise de 0.01 g/kg de poids corporel (PC) à 0.5 g/kg PC, ou de 0.5 g / kg PC à 5 g / kg PC,
ou de 0.01 g/kg PC à 0.05 g/kg PC, ou de 0.05 g/kg PC à 0.1 g/kg PC, ou de 0.1 g/kg PC à 0.5 g/kg PC, ou de 0.5 g/kg PC à 1 g/kg PC, ou de 1 g/kg PC à 3 g/kg PC, ou de 3 g/kg PC à 6 g/kg PC.

Ledit polluant peut être choisi parmi un PCB ou un mélange de PCBs appartenant au groupe des PCBs « dioxin-like », en particulier choisi parmi le PCB 126 (3,3',4,4',5-pentachlorobiphenyl), le PCB 118 (2,3,4,4',5-pentachlorobiphenyl), et/ou des PCBs « non dioxin-like » en particulier choisi parmi le PCB 28 (2,4,4'-Trichlorobiphenyl), le PCB 52 (2,2',5,5'-tetrachloro-1,1'-Biphenyl), le PCB 101 (2,2',4,5,5'-Pentachlorobiphenyl), le PCB 138 (2,2',3,4,4',5'-hexachlorobiphenyl), le PCB 153 (2,3',4,4',5-Pentachlorobiphenyl), le PCB 180 (2,2',3,4,4',5,5'-heptachlorobiphenyl), ou des dioxines ou des pesticides.

Ladite utilisation peut être réalisée chez des sujets humains ou animaux dont les tissus contiennent des PCBs, notamment chez des sujets humains dont les tissus adipeux contiennent des PCBs à une dose allant de 14 à 1700 ng/g.

Ladite utilisation peut être réalisée chez des sujets humains dont le sérum contient des PCBs à une dose allant de 0.9 à 1.5 ng/ml, notamment de 5.4 à 9 ng/ml dans des situations d'exposition particulière et notamment de 900 à 1500 ng/ml lors d'une exposition professionnelle. En particulier, ladite utilisation peut être réalisée chez des sujets humains dont le lait maternel contient des PCBs à une dose allant de 238 à 271 ng/g et notamment 1090 ng/g dans des situations d'exposition particulière.

Selon un mode de réalisation particulier, le polluant est administré à des doses allant de 3 ng/kg PC/jour à 6 mg/kg PC/jour,
notamment de façon chronique, notamment quotidiennement et en particulier à des doses allant de 3 à 50 ng/kg PC/jour, et notamment de 10 à 40 ng/kg PC/jour, plus particulièrement de 20 à 30 ng/kg/jour,
ou notamment de façon aiguë, à des doses supérieures à 1 µg/kg PC/jour, notamment de 1 mg/kg PC/jour à 3 000 mg/kg PC/jour, plus particulièrement de 500 µg/kg PC/jour à 6 mg/kg PC/jour.

Selon, un mode de réalisation particulier, ladite céréale peut être choisie parmi les différentes variétés de blé du genre *Triticum* (blé tendre, blé dur, blé de Khorasan...), l'épeautre, l'orge, l'avoine, le millet, le maïs, le riz, le seigle, le sarrasin, le quinoa, l'amarante, le sésame, le triticale, ladite céréale étant en particulier le blé, ou un mélange de ces céréales.

Ladite prévention ou traitement peut avoir lieu chez l'homme ou chez les animaux, notamment les animaux de compagnie ou de rente.

La toxicité hépatique et cérébrale peut être provoquées par un stress oxydant, ou la toxicité hépatique et cérébrale est provoquée par un mécanisme non oxydant, ou la toxicité hépatique et cérébrale est provoquées exclusivement par un mécanisme non oxydant, ou la toxicité hépatique et cérébrale est provoquées non exclusivement par un mécanisme non oxydant, ou la toxicité hépatique et cérébrale est provoquées par un stress oxydant et/ou un mécanisme non oxydant.

La toxicité hépatique et cérébrale peut être provoquée exclusivement par un mécanisme non oxydant, la toxicité hépatique et cérébrale étant l'augmentation du rapport oméga 6 sur oméga 3 en comparaison du rapport oméga 6 sur oméga 3 égal à 5.

La toxicité hépatique et cérébrale peut être provoquée par un stress oxydant, la toxicité hépatique et cérébrale étant l'augmentation de la peroxydation lipidique dans le foie et le cerveau.

La toxicité hépatique et cérébrale peut être provoquée par un stress oxydant et/ou un mécanisme non oxydant, la toxicité hépatique et cérébrale étant la diminution de l'activité de la citrate synthase, et/ou la perturbation de la chaine respiratoire mitochondriale dans le foie, et/ou la perturbation de l'activité des complexes de la chaine respiratoire mitochondriale dans le cerveau, et/ou l'augmentation de l'activité des transaminases dans le plasma.

La dose unitaire d'aleurone de ladite composition peut être comprise de 0.7 g à 200 g, en particulier 0.7 g à 100 g ou comprise 0.6 g à 180 g.

La présente invention a également pour objet une composition alimentaire ou nutraceutique pour son utilisation pour inhiber la toxicité hépatique et cérébrale due aux polluants, ladite composition comprenant une quantité d'aleurone de 0.7 g à 200 g en particulier 0.7 g à 100 g, ou comprenant une quantité d'aleurone de 0.6 g à 180 g.

Les Inventeurs ont trouvé de manière surprenante que la couche à aleurone présente dans les céréales pouvait réduire la vulnérabilité de l'organisme à ces polluants, et/ou l'en protéger. Les termes « aleurone » et « couche à aleurone » désignent respectivement la matière protéique de réserve et l'assise de l'albumen formée par des grains de ladite matière protéique de réserve, qui sont toutes deux présentes dans les graines.
Une composition comprenant « tout ou partie de la couche à aleurone » signifie que la composition peut comprendre l'ensemble de la couche à aleurone présente dans la graine ou seulement une fraction de cette couche.
Le terme « altérations » désigne tout impact (effets délétères) sur la santé des êtres vivants. Il englobe aussi bien des effets cancérigènes, des atteintes à la fertilité, des perturbations du système endocrinien que des perturbations des systèmes immunitaires et nerveux, notamment du cerveau.
Les polluants au sens large correspondent aux agents physiques, chimiques ou biologiques d'origine naturelle ou non, susceptibles d'avoir des effets délétères sur les organismes vivants, même s'ils sont présents dans le milieu ou l'organisme à des niveaux inférieurs au seuil de nocivité.
Parmi eux, les polluants organiques persistants (POP) sont des substances chimiques persistantes, qui s'accumulent dans les tissus adipeux, se propagent dans la chaîne alimentaire et sont nocives pour la santé et l'environnement.
Les pesticides sont des substances chimiques utilisées pour lutter contre des organismes considérés comme nuisibles et sont nocifs pour la santé et l'environnement. Ils s'accumulent, selon le pesticide considéré, dans de nombreux tissus du corps humain comme par exemple les tissus adipeux, les tissus du cerveau, le sang, les différents organes, le lait maternel,...

Les métaux lourds sont des éléments métalliques naturels dont la masse volumique est supérieure à 5 g/cm3. Les métaux les plus dangereux pour l'homme sont l'aluminium, l'arsenic, le cadmium, le chrome, le nickel, le cuivre, l'étain, le fer, le manganèse, le mercure, le plomb et le zinc. Les métaux lourds s'accumulent dans les organismes vivants et ont des effets toxiques à court et long terme. Selon le métal lourd considéré, l'accumulation a lieu dans différents tissus humains. Par exemple l'aluminium, le mercure et le plomb sont des neurotoxiques, le cadmium et le cuivre s'accumulent, entre autre, dans les reins et le chrome cause troubles respiratoires et inflammation des muqueuses. Plus généralement ils s'accumulent dans différents organes et portent atteinte au système respiratoire, reproductif, digestif et nerveux.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant, ledit polluant étant contenu dans un aliment.

La présente invention a pour objet une composition comprenant tout ou partie de la couche d'aleurone d'une céréale pour son utilisation dans la prévention ou le traitement de la toxicité hépatique et cérébrale induite par au moins un polluant, notamment un polluant organique persistant, ledit polluant étant contenu dans un aliment.

La présente invention a pour objet une composition comprenant tout ou partie de la couche à aleurone d'une céréale pour son utilisation dans la prévention ou le traitement de la toxicité hépatique et cérébrale induite par au moins un polluant, notamment un pesticide, ledit polluant étant contenu dans un aliment.

La présente invention a pour objet une composition comprenant tout ou partie de la couche à aleurone d'une céréale pour son utilisation dans la prévention ou le traitement de la toxicité hépatique et cérébrale induite par au moins un polluant, notamment un métal lourd, ledit polluant étant contenu dans un aliment.

Le terme « aliment » comprend tout aussi bien les aliments d'origine animale, végétale, voire fongique, bactérienne ou minérale, et qu'ils soient sous forme liquide ou solide, susceptibles d'être consommés par l'homme ou l'animal.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant, ledit polluant appartenant au groupe constitué des PCBs, des dioxines, des pesticides et des métaux lourds.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant, ledit polluant appartenant au groupe constitué des PCBs, des dioxines, des pesticides et des métaux lourds.

Selon un mode de réalisation particulier, ledit métal lourd appartient au groupe constitué de l'aluminium, de l'arsenic, du cadmium, du chrome, du nickel, du cuivre, de l'étain, du fer, du manganèse, du mercure, du plomb et du zinc.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant, en particulier pour son utilisation dans la prévention ou le traitement de la toxicité hépatique et cérébrale induite par au moins un polluant, notamment un polluant organique persistant.

Selon un mode de réalisation particulier le polluant est choisi parmi un PCB ou un mélange de PCBs appartenant au groupe des PCBs « dioxin-like », en particulier choisi parmi le PCB 126 (3,3',4,4',5-pentachlorobiphenyl), le PCB 118 (2,3,4,4',5-pentachlorobiphenyl), et/ou des PCBs « non dioxin-like » en particulier choisi parmi le PCB 28 (2,4,4'-Trichlorobiphenyl), le PCB 52 (2,2',5,5'-tetrachloro-1,1'-Biphenyl), le PCB 101 (2,2',4,5,5'-Pentachlorobiphenyl), le PCB 138 (2,2',3,4,4',5'-hexachlorobiphenyl), le PCB 153 (2,3',4,4',5-Pentachlorobiphenyl), le PCB 180 (2,2',3,4,4',5,5'-heptachlorobiphenyl) ou des dioxines ou des pesticides.

Le terme « mélange de PCBs » signifie l'association simultanée d'au moins deux PCBs de formule chimique différente.

Selon une étude de l'Agency for Toxic Substances and Disease Registry (ATSDR, 2000) (1), le taux moyen de PCBs varie dans l'organisme et peut aller selon les populations, de 14 à 1700 ng/g de lipides dans le tissu adipeux, notamment de 14 ng/g à 50 ng/g, 50 ng/g à 100 ng/g, 100 ng/g à 300 ng/g, 300 ng/g à 500 ng/g, 500 ng/g à 700 ng/g, 700 ng/g à 900 ng/g, 900 ng/g à 1100 ng/g, 1100 ng/g à 1300 ng/g, 1300 ng/g à 1500 ng/g ou 1500 ng/g à 1700 ng/g.
Dans le sérum, l'imprégnation varie de 0.9 à 1.5 ng/ml mais peut monter jusqu'à 6 ng/ml chez les forts consommateurs de poissons, notamment de 0.9 ng/ml à 1 ng/ml, 1 ng/ml à 1.5 ng/ml, 1.5 ng/ml à 2 ng/ml, 2 ng/ml à 2.5 ng/ml, 2.5 ng/ml à 3 ng/ml, 3 ng/ml à 3.5 ng/ml, 3.5 ng/ml à 4 ng/ml, 4 ng/ml à 4.5 ng/ml, 4.5 ng/ml à 5 ng/ml, 5 ng/ml à 5.5 ng/ml ou 5.5 ng/ml à 6 ng/ml. L'exposition professionnelle peut entrainer des taux multipliés par 1000 par rapport à la population générale, notamment de 900 ng/ml à 1000 ng/ml, 1000 ng/ml à 1100 ng/ml, 1100 à 1200 ng/ml, 1200 ng/ml à 1300 ng/ml, 1300 ng/ml à 1400 ng/ml ou 1400 ng/ml à 1500 ng/ml. Dans le lait maternel, l'imprégnation varie en moyenne de 238 à 271 ng/g de lipide mais les taux peuvent monter jusqu'à 1090 ng/g dans des situations d'exposition particulière, notamment de 238 ng/g à 250 ng/g, 250 ng/g à 300 ng/g, 300 ng/g à 350 ng/g, 350 ng/g à 400 ng/g, 400 ng/g à 450 ng/g, 450 ng/g à 500 ng/g, 500 ng/g à 550 ng/g, 550 ng/g à 600 ng/g, 600 ng/g à 650 ng/g, 650 ng/g à 700 ng/g, 700 ng/g à 750 ng/g, 750 ng/g à 800 ng/g, 800 ng/g à 850 ng/g, 850 ng/g à 900 ng/g, 900 ng/g à 950 ng/g, 950 ng/g à 1000 ng/g, 1000 ng/g à 1050 ng/g ou 1050 ng/g à 1090 ng/g.
Ce taux de PCBS est présent dans l'organisme avant toute administration d'une composition comprenant tout ou une partie de la couche à aleurone d'une céréale.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, chez des sujets humains ou animaux dont les tissus contiennent ledit polluant.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un PCB, chez des sujets humains ou animaux dont les tissus contiennent ledit PCB.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un pesticide, chez des sujets humains ou animaux dont les tissus contiennent ledit pesticide.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un métal lourd, chez des sujets humains ou animaux dont les tissus contiennent ledit métal lourd.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par des PCBs, chez des sujets humains dont les tissus adipeux contiennent lesdits PCBs à une dose allant de 14 à 1700 ng/g, notamment de 14 ng/g à 50 ng/g, 50 ng/g à 100 ng/g, 100 ng/g à 300 ng/g, 300 ng/g à 500 ng/g, 500 ng/g à 700 ng/g, 700 ng/g à 900 ng/g, 900 ng/g à 1100 ng/g, 1100 ng/g à 1300 ng/g, 1300 ng/g à 1500 ng/g ou 1500 ng/g à 1700 ng/g.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par des PCBs, chez des sujets humains dont le sérum contient lesdits PCBs à une dose allant de 0.9 à 1.5 ng/ml, notamment de 0.9 ng/ml à 1 ng/ml, 1 ng/ml à 1.5 ng/ml, 1.5 ng/ml à 2 ng/ml, 2 ng/ml à 2.5 ng/ml, 2.5 ng/ml à 3 ng/ml, 3 ng/ml à 3.5 ng/ml, 3.5 ng/ml à 4 ng/ml, 4 ng/ml à 4.5 ng/ml, 4.5 ng/ml à 5 ng/ml, 5 ng/ml à 5.5 ng/ml ou 5.5 ng/ml à 6 ng/ml, et plus particulièrement de 5.4 à 9 ng/ml dans des situations d'exposition particulière et de 900 à 1500 ng/ml et notamment de 900 ng/ml à 1000 ng/ml, 1000 ng/ml à 1100 ng/ml, 1100 ng/ml à 1200 ng/ml, 1200 ng/ml à 1300 ng/ml, 1300 ng/ml à 1400 ng/ml ou 1400 ng/ml à 1500 ng/ml lors d'une exposition professionnelle.

On entend par « exposition professionnelle » une exposition qui a lieu dans le cadre d'une activité professionnelle. Il s'agit donc d'une exposition chronique avec des doses plus ou moins fortes conduisant à des accumulations importantes dans les tissus.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par des PCBs, chez des sujets humains dont le lait maternel contient lesdits PCBs à une dose allant de 238 à 271 ng/g et plus particulièrement 1090 ng/g, notamment de 238 ng/g à 250 ng/g, 250 ng/g à 300 ng/g, 300 ng/g à 350 ng/g, 350 ng/g à 400 ng/g, 400 ng/g à 450 ng/g, 450 ng/g à 500 ng/g, 500 ng/g à 550 ng/g, 550 ng/g à 600 ng/g, 600 ng/g à 650 ng/g, 650 ng/g à 700 ng/g, 700 ng/g à 750 ng/g, 750 ng/g à 800 ng/g, 800 ng/g à 850 ng/g, 850 ng/g à 900 ng/g, 900 ng/g à 950 ng/g, 950 ng/g à 1000 ng/g, 1000 ng/g à 1050 ng/g ou 1050 ng/g à 1090 ng/g dans des situations d'exposition particulière.

On entend par « situations d'expositions particulières » des situations particulières induisant des expositions fortes. Par exemple, des personnes qui sont exposées de façon chronique et importante parce qu'elles habitent sur une zone très contaminée ou parce qu'elles habitent près d'une zone avec des émissions particulièrement importante ou parce qu'elles consomment des produits locaux fortement contaminées. Cela peut aussi inclure des personnes ayant subi une exposition accidentelle.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
ledit polluant étant administré à des doses allant de 3 ng/kg Poids Corporel (PC)/jour à 6 mg/kg Poids Corporel (PC)/jour.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
ledit polluant étant administré de façon chronique, notamment quotidiennement.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant ou un pesticide ou un métal lourd,
ledit polluant étant administré de façon chronique, notamment quotidiennement.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant, dans laquelle le polluant est administré de façon chronique, à des doses allant de 3 à 50 ng/kg PC/jour, et notamment de 10 à 40 ng/kg PC/jour, plus particulièrement de 20 à 30 ng/kg PC/jour.
Une administration chronique correspond à l'administration d'un polluant à des doses de l'ordre du ng, mais une administration répétée ou continue dans le temps, notamment tous les jours, et notamment plusieurs fois par jour.

Les PCBs sont dosés par chromatographie gazeuse couplée à de la spectrométrie de masse haute résolution.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
dans laquelle le polluant est administré de façon aiguë, à des doses supérieures à 1 µg/kg PC/jour, notamment de 1 mg/kg PC/jour à 3000 mg/kg PC/jour selon le PCB utilisé (unique ou en mélange), plus particulièrement de 500 µg/kg PC/jour à 6 mg/kg PC/jour, en fonction du ou des PCBs utilisées.

Une administration aiguë d'un polluant correspond à l'administration du polluant à des doses allant de quelques µg à plusieurs mg, soit au moins 1000 fois supérieures à des doses d'une administration chronique. Une telle administration aiguë peut survenir lors d'événements accidentels.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
dans laquelle l'aliment contenant le polluant appartient aux aliments suivants : viandes, œufs, poissons, produits laitiers (laits, beurre, fromages), produits végétaux, ainsi que les aliments à base de produits de cette liste.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant ou un pesticide ou un métal lourd,
dans laquelle l'aliment contenant le polluant appartient aux aliments suivants : viandes, œufs, poissons, produits laitiers (laits, beurre, fromages), produits végétaux, ainsi que les aliments à base de produits de cette liste.

En effet, d'après l'AFSSA, les poissons contribuent à la contamination aux PCBs à 49% chez les enfants, 32 et 36% pour les femmes en âge de procréer et les adultes, les viandes contribuent de 16 à 31%, les produits laitiers (laits, beurre, fromages) contribuent de 14 à 27%, les produits végétaux contribuent de 5 à 12%, les œufs contribuent à 7,5% chez le seul groupe des enfants (AFSSA, 2006, Etablissement de teneurs maximales pertinentes en polychlorobiphényles qui ne sont pas de type dioxine (PCB « non dioxin-like », PCB, NDL) dans divers aliments. Afssa-Saisine n°2006-SA-0305, 1-28).

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
ladite céréale étant choisie parmi les différentes variétés de blé du genre *Triticum* (blé tendre, blé dur, blé de Khorasan...), l'épeautre, l'orge, l'avoine, le millet, le maïs, le riz, le seigle, le sarrasin, le quinoa, l'amarante, le sésame, le triticale, ladite céréale étant en particulier le blé, ou un mélange de ces céréales.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant ou un pesticide ou un métal lourd,
ladite céréale étant choisie parmi les différentes variétés de blé du genre *Triticum* (blé tendre, blé dur, blé de Khorasan...), l'épeautre, l'orge, l'avoine, le millet, le maïs, le riz, le seigle, le sarrasin, le quinoa, l'amarante, le sésame, le triticale, ladite céréale étant en particulier le blé, ou un mélange de ces céréales.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
ladite prévention ou traitement ayant lieu chez l'Homme.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant ou un pesticide ou un métal lourd,
ladite prévention ou traitement ayant lieu chez l'Homme.

De par les caractères de persistance dans le milieu et de bioaccumulation dans la chaine alimentaire des polluants, l'Homme est soumis à une exposition quotidienne aux polluants, notamment aux polluants organiques persistants, particulièrement par le biais de son alimentation.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
ladite prévention ou traitement ayant lieu chez l'animal, notamment l'animal de compagnie ou l'animal de rente.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant ou un pesticide ou un métal lourd,
ladite prévention ou traitement ayant lieu chez l'animal, notamment l'animal de compagnie ou l'animal de rente.

De par leurs caractères de persistance dans le milieu, de bioaccumulation dans la chaine alimentaire et de dissémination sur de longues distances, les polluants, notamment les polluants organiques persistants, entrainent la contamination de l'alimentation animale (sols, végétaux) et de l'eau, et donc la contamination des animaux ainsi que des poissons.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations étant provoquées par un stress oxydant.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant ou un pesticide ou un métal lourd,
lesdites altérations étant provoquées par un stress oxydant.

Le terme de « stress oxydant » correspond à un type d'agression des constituants de la cellule par les espèces réactives d'oxygène.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations étant provoquées par un mécanisme non oxydant.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant ou un pesticide ou un métal lourd,
lesdites altérations étant provoquées par un mécanisme non oxydant.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations étant provoquées exclusivement par un mécanisme non oxydant.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant ou un pesticide ou un métal lourd,
lesdites altérations étant provoquées exclusivement par un mécanisme non oxydant.
Ces altérations sont provoquées exclusivement par un stress oxydant et par aucun autre mécanisme.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations étant provoquées non exclusivement par un mécanisme non oxydant.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant ou un pesticide ou un métal lourd,
lesdites altérations étant provoquées non exclusivement par un mécanisme non oxydant.

Ces altérations ne sont pas provoquées exclusivement par un mécanisme non oxydant, des stress d'autres natures peuvent également intervenir.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations étant provoquées par un mécanisme non oxydant et/ou un stress oxydant.

Selon un mode de réalisation particulier, la présente invention a pour objet une composition comprenant tout ou une partie de la couche à aleurone d'une céréale pour son utilisation dans la prévention ou le traitement de la toxicité hépatique et cérébrale induite par au moins un polluant, notamment un polluant organique persistant ou un pesticide ou un métal lourd,
lesdites altérations étant provoquées par un mécanisme non oxydant et/ou un stress oxydant.

Ces altérations sont provoquées par un mécanisme non oxydant et/ou par un stress oxydant.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations étant l'augmentation du rapport oméga 6 sur oméga 3 en comparaison du rapport oméga 6 sur oméga 3 égal à 5.

L'augmentation du rapport oméga 6 sur oméga 3 est une altération induite par les PCBs qui est provoquée exclusivement par un mécanisme non oxydant.
Les oméga-3 et oméga-6 sont classés comme acides gras essentiels, et interviennent dans divers processus physiologiques. L'AFSSA préconise un rapport oméga 6 sur oméga 3 égal à 5. Il a été montré que l'augmentation du rapport oméga 6 sur oméga 3 est défavorable et entraine entre autres effets, une augmentation des risques cardiovasculaires, des risques de cancer, des risques d'obésité et de troubles métabolique (De Lorgeril et al., 2012, New insights into the health effects of dietary saturated and omega 6 and omega 3 polyunsaturated fatty acids. BMC Med., 21, 10-50) (Muhlhausler et al., 2013, Omega 6 polyunsaturated fatty acids and the early origins of obesity. Curr. Opin. Endocrinol Diabetes Obes. 20 (1), 56-61).

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations se manifestant par une augmentation de la peroxydation lipidique dans le foie.

L'augmentation de la peroxydation lipidique dans le foie est une altération induite par les PCBs et est liée au stress oxydant provoqué par les PCBs dans les cellules hépatiques.
Une administration de PCBs peut engendrer un stress oxydant au niveau des cellules de l'organisme, et provoquer des altérations telles que la peroxydation des lipides, qui est traduite par l'augmentation des substances réactives à l'acide barbiturique (TBARs) tel que le malonedialdéhyde (MBA) issu de la peroxydation lipidique suite à un stress oxydant (exemple 8).

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations se manifestant par une augmentation de la peroxydation lipidique dans le cerveau.
L'augmentation de la peroxydation lipidique dans le cerveau est une altération induite par les PCBs et liée au stress oxydant provoqué par les PCBs dans les cellules du cerveau.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations étant la diminution de l'activité de la citrate synthase.
Cette diminution de l'activité de la citrate synthase est une altération provoquée par un stress oxydant et/ou un mécanisme non oxydant.
L'ensemble des altérations causées par les PCBs ne résultent pas seulement de l'existence d'un stress oxydant. Les PCBs induisent tout aussi bien des altérations dues à un mécanisme non oxydant.
En effet, les 2 familles de PCBs n'ont pas les mêmes mécanismes d'action. Les PCBs-DL se fixent sur le récepteur Ah (Aryl hydrocarbon) et vont induire des modifications d'expression de gènes, alors que les PCB-NDL ne se fixent pas à ce même récepteur. Les PCBs peuvent également se fixer à d'autres types de récepteurs, tels que les récepteurs de la ryanodine (RyR) impliqués dans les effets immunotoxiques et neurotoxiques, les récepteurs de la transthyrétine (TTR), impliqués dans les effets antithyroïdiens, les récepteurs aux oestrogènes (ER) impliqués dans les perturbations œstrogéniques. Les PCBs ont également un effet inhibiteur sur les communications intercellulaires qui les implique dans un mécanisme de promotion tumorale. Ainsi, l'ensemble des altérations observées dans l'organisme ne dépendent pas exclusivement de la présence d'un stress oxydant.
Par ailleurs, en se référant à l'exemple 11 sur la diminution de l'activité de la citrate synthase dans le cerveau, à l'exemple 8 sur l'augmentation de l'activité des transaminases notamment de l'aspartate aminotransférase plasmique et à l'exemple 10 sur la diminution de l'activité des complexes II et III de la chaine respiratoire du cerveau, ces altérations causées par les PCBs se trouvent restitués à leur état basal (niveau des contrôles) lors de la prise d'aleurone. Ces restitutions ne peuvent pas s'expliquer uniquement par la seule action antioxydante de l'acide férulique en réponse au déclenchement d'un stress oxydant En effet, bien qu'étant le composé phénolique majoritaire dans l'aleurone, l'acide férulique possède une faible activité antioxydante par rapport aux molécules testées dans cette étude (Kancheva et al., 2012, Antiradical and antioxidant activities of new bio-antioxydants. Biochimie. 94(2), 403-15). Les résultats obtenus sur ces altérations mettent donc en évidence des propriétés propres à l'aleurone lors de son administration dans le cas d'altérations causées par les PCBs.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations se manifestant par une perturbation de la chaine respiratoire mitochondriale, dans le foie, notamment en réduisant la respiration des mitochondries en conditions phosphorylantes, et ce quel que soit le substrat de respiration.
Cette perturbation de la chaine respiratoire mitochondriale dans le foie, notamment par la réduction de la respiration des mitochondries, est une altération provoquée par un stress oxydant et/ou un mécanisme non oxydant dans les cellules hépatiques.

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations se manifestant par une perturbation de l'activité des complexes de la chaine respiratoire mitochondriale, dans le cerveau, notamment en diminuant l'activité des complexes II et III de la chaine respiratoire.
Cette perturbation de l'activité des complexes de la chaine respiratoire mitochondriale dans le cerveau est une altération provoquée par un stress oxydant et/ou un mécanisme non oxydant dans le cerveau.

Dans la présente invention, les inventeurs ont démontré que les PCBs, même apportés à des doses faibles c'est-à-dire des doses réalistes et représentatives des doses auxquelles un individu est soumis quotidiennement, perturbent de manière significative la fonction mitochondriale dans le foie mais également l'activité de certains complexes de la chaine respiratoire du cerveau.

La chaîne respiratoire mitochondriale a pour rôle essentiel la synthèse d'ATP nécessaire à toutes les cellules de l'organisme. Cette synthèse se fait à partir de cinq complexes multi-enzymatiques localisés dans la membrane interne de la mitochondrie. Ces 5 ensembles de protéines et de coenzymes sont impliqués dans les oxydations phosphorylantes de la chaîne respiratoire. Les 4 premiers complexes (I, II, III et IV) interviennent dans le transport des électrons et le cinquième (V) intervient dans la synthèse d'ATP. Le complexe II correspond au complexe protéique : succinate-ubiquinone oxydoréductase, le complexe III correspond au complexe protéique : ubiquinol-cytochrome C oxydoréductase.

Au niveau hépatique, les Inventeurs ont mis en évidence que l'aleurone pouvait prévenir l'augmentation des transaminases, le stress oxydant, les altérations mitochondriales ainsi que l'augmentation du rapport omega 6/omega 3 induites par des polluants organiques persistants. Dans le foie, l'ensemble de ces altérations peuvent être associées à toutes pathologies hépatiques liées à une cytolyse hépatique telles que :
Stéatose Hépatique Non Alcoolique (NASH), fibrose, cirrhose, hépatites virales, hépatites toxiques, hépatite médicamenteuses, hépatite chronique persistante, hépatite chronique active : dialysés, immunosuppresseurs, hémopathies malignes, carcinome hépatocellulaire, transplantation hépatique, lithiase biliaire, hémochromatose, hémosidérose, maladies de Gaucher, maladie de Nieman Pick, glycogénoses.

Il est à noter également que l'augmentation des transaminases est un signe précoce de toxicité hépatique pouvant être induite par les polluants mais également par les médicaments, l'alcool...

Au niveau du cerveau, les Inventeurs ont mis en évidence que l'aleurone pouvait prévenir l'augmentation du stress oxydant et des altérations mitochondriales induites par les polluants organiques persistants. Dans le cerveau, ces altérations sont des évènements précoces dans l'apparition de maladies liées au vieillissement (Kong et al., 2014) comme l'ensemble des maladies neuro-dégénératives telles que : maladie d'Alzheimer (Picone et al., 2014), maladie de Parkinson (Camilleri and Vassallo, 2014; Gaki and Papavassiliou, 2014), infirmité motrice cérébrale, Ataxie spinocérébelleuse, chorée de Huntington... , troubles cognitifs légers (MCI).

De plus, l'augmentation du stress oxydant dans le cerveau peut également traduire une détérioration des neurotransmetteurs ce qui aurait un impact sur les fonctions cognitives comme l'apprentissage et la mémoire. Enfin, les altérations observées sur le métabolisme énergétique influencent la production d'ATP et donc la croissance neuronale, ce qui pourrait impacter sur le développement cérébral de la descendance lors d'une exposition pendant la gestation.

Au niveau plasmatique, l'augmentation des TBARs induites par les polluants organiques persistants est également associée aux pathologies suivantes : syndrome métabolique, les complications hépatiques du diabète.

Les maladies associées à l'accumulation des pesticides dans le corps humain sont les cancers (prostate, cerveau, estomac, mélanome, leucémies, lymphome non Hodgkiniens, maladie de Hodgkin, myélomes multiples, poumons, colons, pancréas, testicules), maladies neurodégénératives (Parkinson, Alzheimer), malformations congénitales et problèmes de neuro-développement, diabète, problème de dysfonctionnement thyroïdien.

Les maladies associées à l'accumulation de métaux lourds dans le corps humain sont les maladies neurodégénératives (Parkinson, Alzheimer), maladie de Menkes, maladie de Wilson, diabète, syndrome métabolique, insuffisance rénale, sclérose latérale amyotrophique, cancer, autisme, syndrome d'Asperger, saturnisme, maladie de Itai-Itai, hypertension, diminution de la fertilité, maladies cardiovasculaires, athérosclérose, altération des apprentissage et du comportement, trouble de déficit de l'attention /hyperactivité (TDAH).

Selon un mode de réalisation particulier, la présente invention a pour objet une composition comprenant tout ou une partie de la couche à aleurone d'une céréale pour son utilisation dans la prévention ou le traitement des altérations induites par au moins un polluant, notamment un polluant organique persistant,
lesdites altérations se manifestant par une augmentation de l'activité des transaminases
notamment de l'aspartate transaminase, notamment de l'alanine transaminase, dans le plasma. Cette augmentation de l'activité des transaminases dans le plasma est une altération provoquée par un stress oxydant et/ou un mécanisme non oxydant dans le plasma.

La composition selon la présente invention peut être utilisée
à une quantité d'aleurone comprise de 0.01 g/kg PC à 15 g/kg PC, notamment de 0.5 g/kg PC à 5 g/kg PC, plus particulièrement 1 g/kg PC.

Plus particulièrement, la composition selon la présente invention peut être utilisée
à une quantité d'aleurone comprise de 0.0086 g/kg PC à 2.6 g/kg PC, notamment de 0.0086 g/kg à 0.01 g/kg, 0.01 g/kg à 0.05 g/kg, 0.05 g/kg à 0.1 g/kg, 0.1 g/kg à 0.5 g/kg, 0.5 g/kg à 1 g/kg, 1 g/kg à 1.5 g/kg, 1.5 g/kg à 2 g/kg, 2 g/kg à 2.5 g/kg.

Plus particulièrement, la composition selon la présente invention peut être utilisée
à une quantité d'aleurone comprise de 0.01 g/kg PC à 0.05 g/kg PC, 0.05 g/kg PC à 0.1 g/kg PC, 0.1 g/kg PC à 0.5 g/kg PC, 0.5 g/kg PC à 1 g/kg PC, 1 g/kg PC à 3 g/kg PC, 3 g/kg PC à 6 g/kg PC, 6 g/kg PC à 9 g/kg PC, 9 g/kg PC à 12 g/kg PC, 12 g/kg PC à 15 g/kg PC.
La composition définie ci-dessus peut être administrée en une seule ou plusieurs prises.
La dose unitaire d'aleurone à titre d'exemple pour un homme adulte est 0.7 g à 1050 g, notamment 35 g à 350 g, plus particulièrement 70 g.
La dose unitaire d'aleurone à titre d'exemple pour un homme adulte est 0.6 g à 180 g, notamment de 0.6 g à 3.5 g, 3.5 g à 7 g, 7 g à 35 g, 35 g à 70 g, 70 g à 105 g, 105 g à 140 g, 140 g à 180 g.

Plus particulièrement, la dose unitaire d'aleurone à titre d'exemple pour un homme adulte est de 0.7 g à 100 g, 100 g à 200 g, 200 g à 300 g, 300 g à 400 g, 400 g à 500 g, 500 g à 600 g, 600 g à 700 g, 700 g à 800 g, 800 g à 900 g, 900 g à 1 050 g.

La composition selon la présente invention peut être utilisée
à une quantité d'aleurone comprise de 0.01 g/kg PC/jour à 15 g/kg PC/jour, notamment de 0.5 g/kg PC/jour à 5 g/kg PC/jour, plus particulièrement 1 g/kg PC/jour.

Plus particulièrement, la composition selon la présente invention peut être utilisée
à une quantité d'aleurone comprise de 0.0086 g/kg PC/jour à 2.6 g/kg PC/jour, notamment de 0.0086 g/kg PC/jour à 0.01 g/kg PC/jour, 0.01 g/kg PC/jour à 0.05 g/kg PC/jour, 0.05 g/kg PC/jour à 0.1 g/kg PC/jour, 0.1 g/kg PC/jour à 0.5 g/kg PC/jour, 0.5 g/kg PC/jour à 1 g/kg PC/jour, 1 g/kg PC/jour à 1.5 g/kg PC/jour, 1.5 g/kg PC/jour à 2 g/kg PC/jour, 2 g/kg PC/jour à 2.5 g/kg PC/jour.
Plus particulièrement, la composition selon la présente invention peut être utilisée
à une quantité d'aleurone comprise de 0.01 g/kg PC/jour à 0.05 g/kg PC/jour, 0.05 g/kg PC/jour à 0.1 g/kg PC/jour, 0.1 g/kg PC/jour à 0.5 g/kg PC/jour, 0.5 g/kg PC/jour à 1 g/kg PC/jour, 1 g/kg PC/jour à 3 g/kg PC/jour, 3 g/kg PC/jour à 6 g/kg PC/jour, 6 g/kg PC/jour à 9 g/kg PC/jour, 9 g/kg PC/jour à 12 g/kg PC/jour, 12 g/kg PC/jour à 15 g/kg PC/jour.
La composition définie ci-dessus peut être administrée en une seule prise ou plusieurs prises par jour, par exemple en 1 à 5 fois par jour.
La dose unitaire d'aleurone à titre d'exemple pour un homme adulte est 0.7 g/jour à 1050 g/jour, notamment 35 g/jour à 350 g/jour, plus particulièrement 70 g/jour.
La dose unitaire d'aleurone à titre d'exemple pour un homme adulte est 0.6 g/jour à 180 g/jour, notamment de 0.6 g/jour à 3.5 g/jour, 3.5 g/jour à 7 g/jour, 7 g/jour à 35 g/jour, 35 g/jour à 70 g/jour, 70 g/jour à 105 g/jour, 105 g/jour à 140 g/jour, 140 g/jour à 180 g/jour.

Plus particulièrement, la dose unitaire d'aleurone à titre d'exemple pour un homme adulte est de 0.7 g/jour à 100 g/jour, 100 g/jour à 200 g/jour, 200 g/jour à 300 g/jour, 300 g/jour à 400 g/jour, 400 g/jour à 500 g/jour, 500 g/jour à 600 g/jour, 600 g/jour à 700 g/jour, 700 g/jour à 800 g/jour, 800 g/jour à 900 g/jour, 900 g/jour à 1 050 g/jour.

La composition selon la présente invention peut être utilisée
à une quantité d'aleurone dont l'apport énergétique correspondant est de 0.02 kcal/kg PC à 35 kcal/kg PC notamment de 1 kcal/kg PC à 12 kcal/kg PC, plus particulièrement 2.3 kcal/kg PC.

La composition selon la présente invention peut être utilisée
à une quantité d'aleurone dont l'apport énergétique correspondant est de 0.019 kcal/kg PC à 6 kcal/kg PC, notamment de 0.019 kcal/kg PC à 0.1 kcal/kg PC, 0.1 kcal/kg PC à 0.5 kcal/kg PC, 0.5 kcal/kg PC à 1 kcal/kg PC, 1 kcal/kg PC à 1.5 kcal/kg PC, 1.5 kcal/kg PC à 2 kcal/kg PC, 2 kcal/kg PC à 2.5 kcal/kg PC, 2.5 kcal/kg PC à 3 kcal/kg PC, 3 kcal/kg PC à 3.5 kcal/kg PC, 3.5 kcal/kg PC à 4 kcal/kg PC, 4 kcal/kg PC à 4.5 kcal/kg PC, 4.5 kcal/kg PC à 5. 5 kcal/kg PC à 5.5 kcal/kg PC, 5.5 kcal/kg PC à 6 kcal/kg PC.

Plus particulièrement, la composition selon la présente invention peut être utilisée
à une quantité d'aleurone dont l'apport énergétique correspondant est de 0.02 kcal/kg PC à 0.1 kcal/kg PC, de 0.1 kcal/kg PC à 0.5 kcal/kg PC, de 0.5 kcal/kg PC à 1 kcal/kg PC , de 1 kcal/kg PC à 5 kcal/kg PC, de 5 kcal/kg PC à 10 kcal/kg PC, de 10 kcal/kg PC à 15 kcal/kg PC, 15 kcal/kg PC à 20 kcal/kg PC, 20 kcal/kg PC à 25 kcal/kg PC, 25 kcal/kg PC à 30 kcal/kg PC, 30 kcal/kg PC à 35 kcal/kg PC.
La composition définie ci-dessus peut être administrée en une seule ou plusieurs prises.
La dose unitaire d'aleurone à titre d'exemple pour un homme adulte est de 1.4 kcal à 2450 kcal, notamment 70 kcal à 840 kcal, plus particulièrement 161 kcal.
La dose unitaire d'aleurone à titre d'exemple pour un homme adulte est de 1.33 kcal à 420 kcal, notamment de 1.33 kcal à 100 kcal, 100 kcal à 200 kcal, 200 kcal à 300 kcal, 300 kcal à 420 kcal.

Plus particulièrement, la dose unitaire d'aleurone à titre d'exemple pour un homme adulte est de 1.4 kcal à 250 kcal, 250 kcal à 500 kcal, 500 kcal à 1 000 kcal, 1 000 kcal à 1 500 kcal, 1 500 kcal à 2 000 kcal, 2 000 kcal à 2 450 kcal.

La composition selon la présente invention peut être utilisée
à une quantité d'aleurone dont l'apport énergétique correspondant est de 0.02 kcal/kg PC/jour à 35 kcal/kg PC/jour, notamment de 1 kcal/kg PC/jour à 12 kcal/kg PC/jour, plus particulièrement 2.3 kcal/kg PC/jour.

La composition selon la présente invention peut être utilisée
à une quantité d'aleurone dont l'apport énergétique correspondant est de 0.019 kcal/kg PC/jour à 6 kcal/kg PC/jour, notamment de 0.019 kcal/kg PC/jour à 0.1 kcal/kg PC/jour, 0.1 kcal/kg PC/jour à 0.5 kcal/kg PC/jour, 0.5 kcal/kg PC/jour à 1 kcal/kg PC/jour, 1 kcal/kg PC/jour à 1.5 kcal/kg PC/jour, 1.5 kcal/kg PC/jour à 2 kcal/kg PC/jour, 2 kcal/kg PC/jour à 2.5 kcal/kg PC/jour, 2.5 kcal/kg PC/jour à 3 kcal/kg PC/jour, 3 kcal/kg PC/jour à 3.5 kcal/kg PC/jour, 3.5 kcal/kg PC/jour à 4 kcal/kg PC/jour, 4 kcal/kg PC/jour à 4.5 kcal/kg PC/jour, 4.5 kcal/kg PC/jour à 5. 5 kcal/kg PC/jour à 5.5 kcal/kg PC/jour, 5.5 kcal/kg PC/jour à 6 kcal/kg PC/jour.

Plus particulièrement, la composition selon la présente invention peut être utilisée
à une quantité d'aleurone dont l'apport énergétique correspondant est de 0.02 kcal/kg PC/jour à 0.1 kcal/kg PC/jour, de 0.1 kcal/kg PC/jour à 0.5 kcal/kg PC/jour, de 0.5 kcal/kg PC/jour à 1 kcal/kg PC/jour, de 1 kcal/kg PC/jour à 5 kcal/kg PC/jour, de 5 kcal/kg PC/jour à 10 kcal/kg PC/jour, de 10 kcal/kg PC/jour à 15 kcal/kg PC/jour, 15 kcal/kg PC/jour à 20 kcal/kg PC/jour, 20 kcal/kg PC/jour à 25 kcal/kg PC/jour, 25 kcal/kg PC/jour à 30 kcal/kg PC/jour, 30 kcal/kg PC/jour à 35 kcal/kg PC/jour.
La composition définie ci-dessus peut être administrée en une seule prise ou plusieurs prises par jour, par exemple en 1 à 5 fois par jour.
La dose unitaire d'aleurone à titre d'exemple pour un homme adulte est de 1.4 kcal/jour à 2450 kcal/jour.
La dose unitaire d'aleurone à titre d'exemple pour un homme adulte est de 1.33 kcal/jour à 420 kcal/jour, notamment de 1.33 kcal/jour à 100 kcal/jour, 100 kcal/jour à 200 kcal/jour, 200 kcal/jour à 300 kcal/jour, 300 kcal/jour à 420 kcal/jour.

Plus particulièrement, la dose unitaire d'aleurone à titre d'exemple pour un homme adulte est de 1.4 kcal/jour à 250 kcal/jour, 250 kcal/jour à 500 kcal/jour, 500 kcal/jour à 1 000 kcal/jour, 1 000 kcal/jour à 1 500 kcal/jour, 1 500 kcal/jour à 2 000 kcal/jour, 2 000 kcal/jour à 2 450 kcal/jour.

La composition selon la présente invention peut être utilisée, dans le cas d'une administration du polluant de façon chronique,
à une quantité d'aleurone comprise de 0.01 g/kg PC à 5 g/kg PC, notamment de 0.5 g/kg à 3 g/kg PC, plus particulièrement de 1 g/kg PC.

Plus particulièrement, la composition selon la présente invention peut être utilisée
à une quantité d'aleurone comprise de 0.01 g/kg PC à 0.05 g/kg PC, 0.05 g/kg PC à 0.1 g/kg PC, 0.1 g/kg PC à 0.5 g/kg PC, 0.5 g/kg PC à 1 g/kg PC, 1 g/kg PC à 1.5 g/kg PC, 1.5 g/kg PC à 2 g/kg PC, 2 g/kg PC à 2.5 g/kg PC, 2.5 g/kg PC à 3 g/kg PC, 3 g/kg PC à 3.5 g/kg PC, 3.5 g/kg PC à 4 g/kg PC, 4 g/kg PC à 4.5 g/kg PC, 4.5 g/kg PC à 5 g/kg PC.
La composition définie ci-dessus peut être administrée en une seule ou plusieurs prises.
La dose unitaire d'aleurone à titre d'exemple pour un homme adulte est de 0.7 g à 350 g, notamment 35 g à 210 g, plus particulièrement 70 g.
Plus particulièrement, la dose unitaire d'aleurone à titre d'exemple pour un homme adulte est de 0.7 g à 50 g, 50 g à 100 g, 100 g à 150 g, 150 g à 200 g, 200 g à 250 g, 250 g à 300 g, 300 g à 350 g.

La composition selon la présente invention peut être utilisée dans le cas d'une administration du polluant de façon chronique,
à une quantité d'aleurone comprise de O.Olg/kg PC/jour à 5 g/kg PC/jour, notamment de 0.5 g/kg/jour à 3 g/kg PC/jour, plus particulièrement de 1 g/kg PC/jour.
Plus particulièrement, la composition selon la présente invention peut être utilisée à une quantité d'aleurone comprise de 0.01 g/kg PC/jour à 0.05 g/kg PC/jour, 0.05 g/kg PC/jour à 0.1 g/kg PC/jour, 0.1 g/kg PC/jour à 0.5 g/kg PC/jour, 0.5 g/kg PC/jour à 1 g/kg PC/jour, 1 g/kg PC/jour à 1.5 g/kg PC/jour, 1.5 g/kg PC/jour à 2 g/kg PC/jour, 2 g/kg PC/jour à 2.5 g/kg PC/jour, 2.5 g/kg PC/jour à 3 g/kg PC/jour, 3 g/kg PC/jour à 3.5 g/kg PC/jour, 3.5 g/kg PC/jour à 4 g/kg PC/jour, 4 g/kg PC/jour à 4.5 g/kg PC/jour, 4.5 g/kg PC/jour à 5 g/kg PC/jour.
La composition définie ci-dessus peut être administrée en une seule prise ou plusieurs prises par jour, par exemple en 1 à 5 fois par jour.
La dose unitaire d'aleurone à titre d'exemple pour un homme adulte est de 0.7 g/jour à 350 g/jour, notamment 35 g/jour à 210 g/jour, plus particulièrement 70 g/jour.
Plus particulièrement, la dose unitaire d'aleurone à titre d'exemple pour un homme adulte est de 0.7 g/jour à 50 g/jour, 50 g/jour à 100 g/jour, 100 g/jour à 150 g/jour, 150 g/jour à 200 g/jour, 200 g/jour à 250 g/jour, 250 g/jour à 300 g/jour, 300 g/jour à 350 g/jour.

La composition selon la présente invention peut être utilisée dans le cas d'une administration du polluant de façon chronique,
à une quantité d'aleurone dont l'apport énergétique correspondant est de 0.02 kcal/kg PC à 12 kcal/kg PC, notamment de 1 kcal/kg PC à 7 kcal/kg PC, plus particulièrement 2.3 kcal/kg PC.

Plus particulièrement, la composition selon la présente invention peut être utilisée
à une quantité d'aleurone dont l'apport énergétique correspondant est de 0.02 kcal/kg PC à 0.1 kcal/kg PC, 0.1 kcal/kg PC à 0.5 kcal/kg PC, de 0.5 kcal/kg PC à 1 kcal/kg PC, de 1 kcal/kg PC à 2 kcal/kg PC, de 2 kcal/kg PC à 3 kcal/kg PC, 3 kcal/kg PC à 4 kcal/kg PC, 4 kcal/kg PC à 5 kcal/kg PC, 5 kcal/kg PC à 6 kcal/kg PC, 6 kcal/kg PC à 7 kcal/kg PC, 7 kcal/kg PC à 8 kcal/kg PC, 8 kcal/kg PC à 9 kcal/kg PC, 9 kcal/kg PC à 10 kcal/kg PC, 10 kcal/kg PC à 11 kcal/kg PC, 11 kcal/kg PC à 12 kcal/kg PC.
La composition définie ci-dessus peut être administrée en une seule ou plusieurs prises.
La dose unitaire d'aleurone à titre d'exemple pour un homme adulte est de 1.4 kcal à 840 kcal. Plus particulièrement, la dose unitaire d'aleurone à titre d'exemple pour un homme adulte est de 1.4 kcal à 100 kcal, 100 kcal à 200 kcal, 200 kcal à 300 kcal, 300 kcal à 400 kcal, 400 kcal à 500 kcal, 500 kcal à 600 kcal, 600 kcal à 700 kcal, 700 kcal à 840 kcal.

La composition selon la présente invention peut être utilisée dans le cas d'une administration du polluant de façon chronique,
à une quantité d'aleurone dont l'apport énergétique correspondant est de 0.02 kcal/kg PC/jour à 12 kcal/kg PC/jour, notamment de 1 kcal/kg PC/jour à 7 kcal/kg PC/jour, plus particulièrement 2.3 kcal/kg PC/jour.

Plus particulièrement, la composition selon la présente invention peut être utilisée
à une quantité d'aleurone dont l'apport énergétique correspondant est de 0.02 kcal/kg PC/jour à 0.1 kcal/kg PC/jour, 0.1 kcal/kg PC/jour à 0.5 kcal/kg PC/jour, de 0.5 kcal/kg PC/jour à 1 kcal/kg PC/jour, de 1 kcal/kg PC/jour à 2 kcal/kg PC/jour, de 2 kcal/kg PC/jour à 3 kcal/kg PC/jour, 3 kcal/kg PC/jour à 4 kcal/kg PC/jour, 4 kcal/kg PC/jour à 5 kcal/kg PC/jour, 5 kcal/kg PC/jour à 6 kcal/kg PC/jour, 6 kcal/kg PC/jour à 7 kcal/kg PC/jour, 7 kcal/kg PC/jour à 8 kcal/kg PC/jour, 8 kcal/kg PC/jour à 9 kcal/kg PC/jour, 9 kcal/kg PC/jour à 10 kcal/kg PC/jour, 10 kcal/kg PC/jour à 11 kcal/kg PC/jour, 11 kcal/kg PC/jour à 12 kcal/kg PC/jour.
La composition définie ci-dessus peut être administrée en une seule prise ou plusieurs prises par jour, par exemple en 1 à 5 fois par jour.
La dose unitaire d'aleurone à titre d'exemple pour un homme adulte est de 1.4 kcal/jour à 840 kcal/jour.
Plus particulièrement, la dose unitaire d'aleurone à titre d'exemple pour un homme adulte est de 1.4 kcal/jour à 100 kcal/jour, 100 kcal/jour à 200 kcal/jour, 200 kcal/jour à 300 kcal/jour, 300 kcal/jour à 400 kcal/jour, 400 kcal/jour à 500 kcal/jour, 500 kcal/jour à 600 kcal/jour, 600 kcal/jour à 700 kcal/jour, 700 kcal/jour à 840 kcal/jour.

La composition selon la présente invention peut être utilisée dans le cas d'une administration du polluant de façon aiguë,
à une quantité d'aleurone d'une valeur supérieure à 5 g/kg PC à 15 g/kg PC, notamment de 6 g/kg PC à 8 g/kg PC, plus particulièrement de 7 g/kg PC.

Plus particulièrement, la composition selon la présente invention peut être utilisée
à une quantité d'aleurone d'une valeur supérieure à 5 g/kg PC à 6 g/kg PC, 6 g/kg PC à 7 g/kg PC, 7 g/kg PC à 8 g/kg PC, 8 g/kg PC à 9 g/kg PC, 9 g/kg PC à 10 g/kg PC, 10 g/kg PC à 11 g/kg PC, 11 g/kg PC à 12 g/kg PC, 12 g/kg PC à 13 g/kg PC, 13 g/kg PC à 14 g/kg PC, 14 g/kg PC à 15 g/kg PC.
La composition définie ci-dessus peut être administrée en une seule ou plusieurs prises.
La dose unitaire d'aleurone à titre d'exemple pour un homme adulte est d'une valeur supérieure à 350 g à 1050 g, notamment 420 g à 560 g, plus particulièrement 490 g.
Plus particulièrement, la dose unitaire d'aleurone à titre d'exemple pour un homme adulte est d'une valeur supérieure à 350 g à 450 g, 450 g à 550 g, 550 g à 650 g, 650 g à 750 g, 750 g à 850 g, 850 g à 950 g, 950 g à 1050 g.

La composition selon la présente invention peut être utilisée dans le cas d'une administration du polluant de façon aiguë,
à une quantité d'aleurone d'une valeur supérieure à 5 g/kg PC/jour à 15 g/kg PC/jour, notamment de 6 g/kg PC/jour à 8 g/kg PC/jour, plus particulièrement de 7 g/kg PC/jour.
Plus particulièrement, la composition selon la présente invention peut être utilisée
à une quantité d'aleurone d'une valeur supérieure à 5 g/kg PC/jour à 6 g/kg PC/jour, 6 g/kg PC/jour à 7 g/kg PC/jour, 7 g/kg PC/jour à 8 g/kg PC/jour, 8 g/kg PC/jour à 9 g/kg PC/jour, 9 g/kg PC/jour à 10 g/kg PC/jour, 10 g/kg PC/jour à 11 g/kg PC/jour, 11 g/kg PC/jour à 12 g/kg PC/jour, 12 g/kg PC/jour à 13 g/kg PC/jour, 13 g/kg PC/jour à 14 g/kg PC/jour, 14 g/kg PC/jour à 15 g/kg PC/jour.
La composition définie ci-dessus peut être administrée en une seule prise ou plusieurs prises par jour, par exemple en 1 à 5 fois par jour.
La dose unitaire d'aleurone à titre d'exemple pour un homme adulte est d'une valeur supérieure à 350 g/jour à 1050 g/jour, notamment 420 g/jour à 560 g/jour, plus particulièrement 490 g/jour.
Plus particulièrement, la dose unitaire d'aleurone à titre d'exemple pour un homme adulte est d'une valeur supérieure à 350 g/jour à 450 g/jour, 450 g/jour à 550 g/jour, 550 g/jour à 650 g/jour, 650 g/jour à 750 g/jour, 750 g/jour à 850 g/jour, 850 g/jour à 950 g/jour, 950 g/jour à 1050 g/jour.

La composition selon la présente invention peut être utilisée dans le cas d'une administration du polluant de façon aiguë,
à une quantité d'aleurone dont l'apport énergétique correspondant est d'une valeur supérieure à 12 kcal/kg PC à 35 kcal/kg PC, notamment 14 kcal/kg PC à 19 kcal/kg PC, plus particulièrement 16 kcal/kg PC.

Plus particulièrement, la composition selon la présente invention peut être utilisée
à une quantité d'aleurone dont l'apport énergétique correspondant est d'une valeur supérieure à 12 kcal/kg PC à 14 kcal/kg PC, de 14 kcal/kg PC à 16 kcal/kg PC, de 16 kcal/kg PC à 18 kcal/kg PC, de 18 kcal/kg PC à 20 kcal/kg PC, 20 kcal/kg PC à 22 kcal/kg PC, 22 kcal/kg PC à 24 kcal/kg PC, 24 kcal/kg PC à 26 kcal/kg PC,26 kcal/kg PC à 28 kcal/kg PC, 28 kcal/kg PC à 30 kcal/kg PC, 30 kcal/kg PC à 32 kcal/kg PC, 32 kcal/kg PC à 35 kcal/kg PC.
La composition définie ci-dessus peut être administrée en une seule ou plusieurs prises.
La dose unitaire d'aleurone à titre d'exemple pour un homme adulte est d'une valeur supérieure à 840 kcal à 2450 kcal, notamment de 980 kcal à 1330 kcal, plus particulièrement 1120 kcal.
Plus particulièrement, la dose unitaire d'aleurone à titre d'exemple pour un homme adulte est d'une valeur supérieure à 840 kcal à 1050 kcal, 1050 kcal à 1250 kcal, 1250 kcal à 1450 kcal, 1450 kcal à 1650 kcal, 1650 kcal à 1850 kcal, 1850 kcal à 2050 kcal, 2050 kcal à 2250 kcal, 2250 kcal à 2450 kcal.

La composition selon la présente invention peut être utilisée dans le cas d'une administration du polluant de façon aiguë,
à une quantité d'aleurone dont l'apport énergétique correspondant est d'une valeur supérieure à 12 kcal/kg PC/jour à 35 kcal/kg PC/jour, notamment 14 kcal/kg PC/jour à 19 kcal/kg PC/jour, plus particulièrement 16 kcal/kg PC/jour.

Plus particulièrement, la composition selon la présente invention peut être utilisée
à une quantité d'aleurone dont l'apport énergétique correspondant est d'une valeur supérieure à 12 kcal/kg PC/jour à 14 kcal/kg PC/jour, de 14 kcal/kg PC/jour à 16 kcal/kg PC/jour, de 16 kcal/kg PC/jour à 18 kcal/kg PC/jour, de 18 kcal/kg PC/jour à 20 kcal/kg PC/jour, 20 kcal/kg PC/jour à 22 kcal/kg PC/jour, 22 kcal/kg PC/jour à 24 kcal/kg PC/jour, 24 kcal/kg PC/jour à 26 kcal/kg PC/jour,26 kcal/kg PC/jour à 28 kcal/kg PC/jour, 28 kcal/kg PC/jour à 30 kcal/kg PC/jour, 30 kcal/kg PC/jour à 32 kcal/kg PC/jour, 32 kcal/kg PC/jour à 35 kcal/kg PC/jour.
La composition définie ci-dessus peut être administrée en une seule prise ou plusieurs prises par jour, par exemple en 1 à 5 fois par jour.
La dose unitaire d'aleurone à titre d'exemple pour un homme adulte est d'une valeur supérieure à 840 kcal/jour à 2450 kcal/jour, notamment de 980 kcal/jour à 1330 kcal/jour, plus particulièrement 1120 kcal/jour.

Plus particulièrement, la dose unitaire d'aleurone à titre d'exemple pour un homme adulte est d'une valeur supérieure à 840 kcal/jour à 1050 kcal/jour, 1050 kcal/jour à 1250 kcal/jour, 1250 kcal/jour à 1450 kcal/jour, 1450 kcal/jour à 1650 kcal/jour, 1650 kcal/jour à 1850 kcal/jour, 1850 kcal/jour à 2050 kcal/jour, 2050 kcal/jour à 2250 kcal/jour, 2250 kcal/jour à 2450 kcal/jour. La composition selon la présente invention peut être utilisée
par voie orale sous forme d'un dosage unitaire d'une à cinq fois par jour avant ou pendant ou après les repas.
La composition selon la présente invention peut être utilisée dans le cas de l'administration du polluant de façon aiguë
par voie orale sous forme d'un dosage unitaire d'une à 5 fois par jour avant ou pendant ou après les repas.

Selon un mode de réalisation particulier, la présente invention a également pour objet l'utilisation d'aleurone pour la préparation d'une composition nutraceutique ou d'un complément alimentaire pour inhiber les altérations dues aux polluants,
ladite composition comprenant une quantité d'aleurone de 0.01 g/kg PC à 15 g/kg PC, notamment de 0.5 g/kg PC à 5 g/kg PC, plus particulièrement 1 g/kg PC.

Selon un mode de réalisation particulier, la présente invention a également pour objet l'utilisation d'aleurone pour la préparation d'une composition nutraceutique ou d'un complément alimentaire pour inhiber la toxicité hépatique et cérébrale due aux polluants,
ladite composition comprenant une quantité d'aleurone de 0.01 g/kg PC à 15 g/kg PC, notamment de 0.5 g/kg PC à 5 g/kg PC, plus particulièrement 1 g/kg PC.

Selon un mode de réalisation particulier, la présente invention a également pour objet l'utilisation d'aleurone pour la préparation d'une composition nutraceutique ou d'un complément alimentaire pour inhiber la toxicité hépatique et cérébrale due aux polluants,
ladite composition comprenant une quantité d'aleurone de 0.0086 g/kg PC à 2.6 g/kg PC, notamment de 0.0086 g/kg PC/jour à 0.01 g/kg PC/jour, 0.01 g/kg PC/jour à 0.05 g/kg PC/jour, 0.05 g/kg PC/jour à 0.1 g/kg PC/jour, 0.1 g/kg PC/jour à 0.5 g/kg PC/jour, 0.5 g/kg PC/jour à 1 g/kg PC/jour, 1 g/kg PC/jour à 1.5 g/kg PC/jour, 1.5 g/kg PC/jour à 2 g/kg PC/jour, 2 g/kg PC/jour à 2.5 g/kg PC/jour.

Plus particulièrement, la présente invention a également pour objet l'utilisation d'aleurone pour la préparation d'une composition nutraceutique ou d'un complément alimentaire,
ladite composition comprenant une quantité d'aleurone de 0.01 g/kg PC à 0.05 g/kg PC, 0.05 g/kg PC à 0.1 g/kg PC, 0.1 g/kg PC à 0.5 g/kg PC, 0.5 g/kg PC à 1 g/kg PC, 1 g/kg PC à 3 g/kg PC, 3 g/kg PC à 6 g/kg PC, 6 g/kg PC à 9 g/kg PC, 9 g/kg PC à 12 g/kg PC, 12 g/kg PC à 15 g/kg PC.
La composition définie ci-dessus peut être administrée en une seule ou plusieurs prises.
La dose unitaire d'aleurone à titre d'exemple pour un homme adulte est 0.7 g à 1050 g, notamment 35 g à 350 g, plus particulièrement 70 g.
La dose unitaire d'aleurone à titre d'exemple pour un homme adulte est 0.6 g à 180 g, notamment de 0.6 g à 3.5 g, 3.5 g à 7 g, 7 g à 35 g, 35 g à 70 g, 70 g à 105 g, 105 g à 140 g, 140 g à 180 g.

Plus particulièrement, la dose unitaire d'aleurone à titre d'exemple pour un homme adulte est de 0.7 g à 100 g, 100 g à 200 g, 200 g à 300 g, 300 g à 400 g, 400 g à 500 g, 500 g à 600 g, 600 g à 700 g, 700 g à 800 g, 800 g à 900 g, 900 g à 1 050 g.

Les inventeurs ont mis en évidence qu'une alimentation enrichie avec de la couche à aleurone peut prévenir la toxicité sur le foie et le cerveau induite par des polluants organiques persistants, tels que les PCBs. Ainsi, afin de réduire les altérations dues à ces polluants, notamment des PCBs, les inventeurs ont développé une composition alimentaire ou nutraceutique.
Les termes « composition nutraceutique ou complément alimentaire » correspondent à une composition contenant au moins un produit fabriqué à partir d'aliments ayant un effet physiologique dans la prévention des maladies et commercialisée sous forme de gélule, de comprimé, de poudre, de potion, de pastilles, de pilules et autres formes similaires, ainsi que les sachets de poudre, les ampoules de liquide, les flacons munis d'un compte-gouttes et les autres formes analogues de préparations liquides ou en poudre destinées à être prises en unités mesurées de faible quantité.

Selon un mode de réalisation particulier, la présente demande a également pour objet l'utilisation de l'aleurone sous la forme d'un ingrédient ajouté dans les aliments, dans les Aliments Destinés à l'Alimentation de Personnes Particulières, dans un complément nutritionnel, chez l'Homme, ainsi que dans les aliments, médicaments, et/ou compléments alimentaires destinés aux animaux, notamment aux animaux de compagnie et animaux de rente.
Selon un mode de réalisation particulier, dans le cas d'une administration du polluant de façon chronique, la présente invention a également pour objet l'utilisation d'aleurone pour la préparation d'une composition nutraceutique ou d'un complément alimentaire pour inhiber les altérations dues aux polluants,
ladite composition comprenant une quantité d'aleurone de 0.01 g/kg PC à 5 g/kg PC, notamment de 0.5 g/kg à 3 g/kg PC, plus particulièrement de 1 g/kg PC.

Plus particulièrement, la présente invention a également pour objet l'utilisation d'aleurone pour la préparation d'une composition nutraceutique ou d'un complément alimentaire,
ladite composition comprenant une quantité d'aleurone de 0.01 g/kg PC à 0.05 g/kg PC, 0.05 g/kg PC à 0.1 g/kg PC, 0.1 g/kg PC à 0.5 g/kg PC, 0.5 g/kg PC à 1 g/kg PC, 1 g/kg PC à 1.5 g/kg PC, 1.5 g/kg PC à 2 g/kg PC, 2 g/kg PC à 2.5 g/kg PC, 2.5 g/kg PC à 3 g/kg PC, 3 g/kg PC à 3.5 g/kg PC, 3.5 g/kg PC à 4 g/kg PC, 4 g/kg PC à 4.5 g/kg PC, 4.5 g/kg PC à 5 g/kg PC.
La composition définie ci-dessus peut être administrée en une seule ou plusieurs prises.
La dose unitaire d'aleurone à titre d'exemple pour un homme adulte est de 0.7 g à 350 g, notamment 35 g à 210 g, plus particulièrement 70 g.
Plus particulièrement, la dose unitaire d'aleurone à titre d'exemple pour un homme adulte est de 0.7 g à 50 g, 50 g à 100 g, 100 g à 150 g, 150 g à 200 g, 200 g à 250 g, 250 g à 300 g, 300 g à 350 g.

Selon un mode de réalisation particulier, dans le cas d'une administration du polluant de façon chronique, la présente invention a également pour objet l'utilisation d'aleurone pour la préparation d'une composition nutraceutique ou d'un complément alimentaire pour inhiber les altérations dues aux polluants,
ladite composition comprenant une quantité d'aleurone de O.Olg/kg PC/jour à 5 g/kg PC/jour, notamment de 0.5 g/kg PC/jour à 3 g/kg PC/jour, plus particulièrement de 1 g/kg PC/jour.

Selon un mode de réalisation particulier, dans le cas d'une administration du polluant de façon chronique, la présente invention a également pour objet l'utilisation d'aleurone pour la préparation d'une composition nutraceutique ou d'un complément alimentaire pour inhiber la toxicité hépatique et cérébrale dues aux polluants,
ladite composition comprenant une quantité d'aleurone de O.Olg/kg PC/jour à 5 g/kg PC/jour, notamment de 0.5 g/kg PC/jour à 3 g/kg PC/jour, plus particulièrement de 1 g/kg PC/jour.

Selon un mode de réalisation particulier, dans le cas d'une administration du polluant de façon chronique, la présente invention a également pour objet l'utilisation d'aleurone pour la préparation d'une composition nutraceutique ou d'un complément alimentaire pour inhiber la toxicité hépatique et cérébrale dues aux polluants,
ladite composition comprenant une quantité d'aleurone de 0.0086 g/kg PC/jour à 2.6 g/kg PC/jour, notamment de 0.0086 g/kg PC/jour à 0.01 g/kg PC/jour, 0.01 g/kg PC/jour à 0.05 g/kg PC/jour, 0.05 g/kg PC/jour à 0.1 g/kg PC/jour, 0.1 g/kg PC/jour à 0.5 g/kg PC/jour, 0.5 g/kg PC/jour à 1 g/kg PC/jour, 1 g/kg PC/jour à 1.5 g/kg PC/jour, 1.5 g/kg PC/jour à 2 g/kg PC/jour, 2 g/kg PC/jour à 2.5 g/kg PC/jour.

Plus particulièrement, la présente invention a également pour objet l'utilisation d'aleurone pour la préparation d'une composition nutraceutique ou d'un complément alimentaire,
ladite composition comprenant une quantité d'aleurone de 0.01 g/kg PC/jour à 0.05 g/kg PC/jour, 0.05 g/kg PC/jour à 0.1 g/kg PC/jour, 0.1 g/kg PC/jour à 0.5 g/kg PC/jour, 0.5 g/kg PC/jour à 1 g/kg PC/jour, 1 g/kg PC/jour à 1.5 g/kg PC/jour, 1.5 g/kg PC/jour à 2 g/kg PC/jour, 2 g/kg PC/jour à 2.5 g/kg PC/jour, 2.5 g/kg PC/jour à 3 g/kg PC/jour, 3 g/kg PC/jour à 3.5 g/kg PC/jour, 3.5 g/kg PC/jour à 4 g/kg PC/jour, 4 g/kg PC/jour à 4.5 g/kg PC/jour, 4.5 g/kg PC/jour à 5 g/kg PC/jour.
La composition définie ci-dessus peut être administrée en une seule ou plusieurs prises par jour, par exemple en 1 à 5 fois par jour.
La dose unitaire d'aleurone à titre d'exemple pour un homme adulte est de 0.7 g/jour à 350 g/jour, notamment 35 g/jour à 210 g/jour, plus particulièrement 70 g/jour.
La dose unitaire d'aleurone à titre d'exemple pour un homme adulte est de 0.6 g/jour à 180 g/jour, notamment de 0.6 g à 3.5 g, 3.5 g à 7 g, 7 g à 35 g, 35 g à 70 g, 70 g à 105 g, 105 g à 140 g, 140 g à 180 g.

Plus particulièrement, la dose unitaire d'aleurone à titre d'exemple pour un homme adulte est de 0.7 g/jour à 50 g/jour, 50 g/jour à 100 g/jour, 100 g/jour à 150 g/jour, 150 g/jour à 200 g/jour, 200 g/jour à 250 g/jour, 250 g/jour à 300 g/jour, 300 g/jour à 350 g/jour.

Selon un mode de réalisation particulier, dans le cas d'une administration du polluant de façon aiguë, la présente invention a également pour objet l'utilisation d'aleurone pour la préparation d'une composition nutraceutique ou d'un complément alimentaire pour inhiber les altérations dues aux polluants,
ladite composition comprenant une quantité d'aleurone d'une valeur supérieure à 5 g/kg PC à 15 g/kg PC, notamment 6 g/kg PC à 8 g/kg PC plus particulièrement de 7 g/kg PC.

Plus particulièrement, la présente invention a également pour objet l'utilisation d'aleurone pour la préparation d'une composition nutraceutique ou d'un complément alimentaire,
ladite composition comprenant une quantité d'aleurone d'une valeur supérieure à 5 g/kg PC à 6 g/kg PC, 6 g/kg PC à 7 g/kg PC, 7 g/kg PC à 8 g/kg PC, 8 g/kg PC à 9 g/kg PC, 9 g/kg PC à 10 g/kg PC, 10 g/kg PC à 11 g/kg PC, 11 g/kg PC à 12 g/kg PC, 12 g/kg PC à 13 g/kg PC, 13 g/kg PC à 14 g/kg PC, 14 g/kg PC à 15 g/kg PC.
La dose unitaire d'aleurone à titre d'exemple pour un homme adulte est d'une valeur supérieure à 350 g à 1050 g, notamment 420 g à 560 g, plus particulièrement 490 g.
Plus particulièrement, la dose unitaire d'aleurone à titre d'exemple pour un homme adulte est d'une valeur supérieure à 350 g à 450 g, 450 g à 550 g, 550 g à 650 g, 650 g à 750 g, 750 g à 850 g, 850 g à 950 g, 950 g à 1050 g.
Selon un mode de réalisation particulier, dans le cas d'une administration du polluant de façon aiguë, la présente invention a également pour objet l'utilisation d'aleurone pour la préparation d'une composition nutraceutique ou d'un complément alimentaire pour inhiber les altérations dues aux polluants,
ladite composition comprenant une quantité d'aleurone d'une valeur supérieure à 5 g/kg PC/jour à 15 g/kg PC/jour, notamment 6 g/kg PC/jour à 8 g/kg PC/jour plus particulièrement de 7 g/kg PC/jour.

Plus particulièrement, la présente invention a également pour objet l'utilisation d'aleurone pour la préparation d'une composition nutraceutique ou d'un complément alimentaire,
ladite composition comprenant une quantité d'aleurone d'une valeur supérieure à 5 g/kg PC/jour à 6 g/kg PC/jour, 6 g/kg PC/jour à 7 g/kg PC/jour, 7 g/kg PC/jour à 8 g/kg PC/jour, 8 g/kg PC/jour à 9 g/kg PC/jour, 9 g/kg PC/jour à 10 g/kg PC/jour, 10 g/kg PC/jour à 11 g/kg PC/jour, 11 g/kg PC/jour à 12 g/kg PC/jour, 12 g/kg PC/jour à 13 g/kg PC/jour, 13 g/kg PC/jour à 14 g/kg PC/jour, 14 g/kg PC/jour à 15 g/kg PC/jour.
La dose unitaire d'aleurone à titre d'exemple pour un homme adulte est d'une valeur supérieure à 350 g/jour à 1050 g/jour, notamment 420 g/jour à 560 g/jour, plus particulièrement 490 g/jour. Plus particulièrement, la dose unitaire d'aleurone à titre d'exemple pour un homme adulte est de 350 g/jour à 450 g/jour, 450 g/jour à 550 g/jour, 550 g/jour à 650 g/jour, 650 g/jour à 750 g/jour, 750 g/jour à 850 g/jour, 850 g/jour à 950 g/jour, 950 g/jour à 1050 g/jour.

Selon un mode de réalisation particulier, la présente invention a également pour objet, une composition alimentaire ou nutraceutique pour inhiber les altérations dues aux polluants,
ladite composition comprenant une quantité d'aleurone de 0.01 g/kg PC à 15 g/kg PC, notamment de 0.5 g/kg PC à 5 g/kg PC, plus particulièrement de 1 g/kg PC.
Plus particulièrement, la présente invention a également pour objet, une composition alimentaire ou nutraceutique,
ladite composition comprenant une quantité d'aleurone de 0.01 g/kg PC à 0.05 g/kg PC, 0.05 g/kg PC à 0.1 g/kg PC, 0.1 g/kg PC à 0.5 g/kg PC, 0.5 g/kg PC à 1 g/kg PC, 1 g/kg PC à 3 g/kg PC, 3 g/kg PC à 6 g/kg PC, 6 g/kg PC à 9 g/kg PC, 9 g/kg PC à 12 g/kg PC, 12 g/kg PC à 15 g/kg PC.

La composition définie ci-dessus peut être administrée en une seule ou plusieurs prises. La dose unitaire d'aleurone à titre d'exemple pour un homme adulte est de 0.7 g à 1050 g, notamment 35 g à 350 g, plus particulièrement 70 g.
Plus particulièrement, la dose unitaire d'aleurone à titre d'exemple pour un homme adulte est de 0.7 g à 100 g, 100 g à 200 g, 200 g à 300 g, 300 g à 400 g, 400 g à 500 g, 500 g à 600 g, 600 g à 700 g, 700 g à 800 g, 800 g à 900 g, 900 g à 1 050 g.

Selon un mode de réalisation particulier, la présente invention a également pour objet, une composition alimentaire ou nutraceutique pour inhiber les altérations dues aux polluants,
ladite composition comprenant une quantité d'aleurone de 0.01 g/kg PC/jour à 15 g/kg PC/jour, notamment de 0.5 g/kg PC/jour à 5 g/kg PC/jour, en particulier de 1 g/kg PC/jour.
Plus particulièrement, la présente invention a également pour objet, une composition alimentaire ou nutraceutique,
ladite composition comprenant une quantité d'aleurone de 0.01 g/kg PC/jour à 0.05 g/kg PC/jour, 0.05 g/kg PC/jour à 0.1 g/kg PC/jour, 0.1 g/kg PC/jour à 0.5 g/kg PC/jour, 0.5 g/kg PC/jour à 1 g/kg PC/jour, 1 g/kg PC/jour à 3 g/kg PC/jour, 3 g/kg PC/jour à 6 g/kg PC/jour, 6 g/kg PC/jour à 9 g/kg PC/jour, 9 g/kg PC/jour à 12 g/kg PC/jour, 12 g/kg PC/jour à 15 g/kg PC/jour.

La composition définie ci-dessus peut être administrée en une seule ou plusieurs prises par jour, par exemple en 1 à 5 fois par jour.
La dose unitaire d'aleurone à titre d'exemple pour un homme adulte est de 0.7 g/jour à 1050 g/jour, notamment 35 g/jour à 350 g/jour, plus particulièrement 70 g/jour.
Plus particulièrement, la dose unitaire d'aleurone à titre d'exemple pour un homme adulte est de 0.7 g/jour à 100 g/jour, 100 g/jour à 200 g/jour, 200 g/jour à 300 g/jour, 300 g/jour à 400 g/jour, 400 g/jour à 500 g/jour, 500 g/jour à 600 g/jour, 600 g/jour à 700 g/jour, 700 g/jour à 800 g/jour, 800 g/jour à 900 g/jour, 900 g/jour à 1 050 g/jour.

Selon un mode de réalisation particulier, dans le cas d'une administration du polluant de façon chronique, la présente invention a également pour objet une composition alimentaire ou nutraceutique, pour inhiber les altérations dues aux polluants,
ladite composition comprenant une quantité d'aleurone de 0.01 g/kg PC à 5 g/kg PC, notamment de 0.5 g/kg à 3 g/kg PC, plus particulièrement de 1 g/kg PC.

Plus particulièrement, la présente invention a également pour objet l'utilisation d'aleurone pour la préparation d'une composition nutraceutique ou d'un complément alimentaire,
ladite composition comprenant une quantité d'aleurone de 0.01 g/kg PC à 0.05 g/kg PC, 0.05 g/kg PC à 0.1 g/kg PC, 0.1 g/kg PC à 0.5 g/kg PC, 0.5 g/kg PC à 1 g/kg PC, 1 g/kg PC à 1.5 g/kg PC, 1.5 g/kg PC à 2 g/kg PC, 2 g/kg PC à 2.5 g/kg PC, 2.5 g/kg PC à 3 g/kg PC, 3 g/kg PC à 3.5 g/kg PC, 3.5 g/kg PC à 4 g/kg PC, 4 g/kg PC à 4.5 g/kg PC, 4.5 g/kg PC à 5 g/kg PC.

Selon un mode de réalisation particulier, dans le cas d'une administration du polluant de façon chronique, la présente invention a également pour objet une composition alimentaire ou nutraceutique, pour inhiber les altérations dues aux polluants,
ladite composition étant sous forme unitaire et comprenant une quantité d'aleurone de 0.7 g à 350 g, notamment de 35 g à 210 g, plus particulièrement de 70 g.

Plus particulièrement, la présente invention à également pour objet une composition alimentaire ou nutraceutique, pour inhiber les altérations dues aux polluants, ladite composition étant sous forme unitaire et comprenant une quantité d'aleurone de 0.7 g à 50 g, 50 g à 100 g, 100 g à 150 g, 150 g à 200 g, 200 g à 250 g, 250 g à 300 g, 300 g à 350 g.

Selon un mode de réalisation particulier, dans le cas d'une administration du polluant de façon aiguë, la présente invention a également pour objet une composition alimentaire ou nutraceutique, pour inhiber les altérations dues aux polluants,
ladite composition comprenant une quantité d'aleurone d'une valeur supérieure à 5 g/kg PC à 15 g/kg PC, notamment 6 g/kg PC à 8 g/kg PC, plus particulièrement de 7 g/kg PC.

Plus particulièrement, la présente invention a également pour objet l'utilisation d'aleurone pour la préparation d'une composition nutraceutique ou d'un complément alimentaire,
ladite composition comprenant une quantité d'aleurone d'une valeur supérieure à 5 g/kg PC à 6 g/kg PC, 6 g/kg PC à 7 g/kg PC, 7 g/kg PC à 8 g/kg PC, 8 g/kg PC à 9 g/kg PC, 9 g/kg PC à 10 g/kg PC, 10 g/kg PC à 11 g/kg PC, 11 g/kg PC à 12 g/kg PC, 12 g/kg PC à 13 g/kg PC, 13 g/kg PC à 14 g/kg PC, 14 g/kg PC à 15 g/kg PC.

Selon un mode de réalisation particulier, dans le cas d'une administration du polluant de façon aiguë, la présente invention a également pour objet une composition alimentaire ou nutraceutique, pour inhiber les altérations dues aux polluants,
ladite composition étant sous forme unitaire et comprenant une quantité d'aleurone d'une valeur supérieure à 350 g à 1050 g, notamment 420 g à 560 g, plus particulièrement de 490 g. Plus particulièrement, la présente invention a également pour objet une composition alimentaire ou nutraceutique,
ladite composition étant sous forme unitaire et comprenant une quantité d'aleurone d'une valeur supérieure à 350 g à 450 g, 450 g à 550 g, 550 g à 650 g, 650 g à 750 g, 750 g à 850 g, 850 g à 950 g, 950 g à 1050 g.

Selon un mode de réalisation particulier, la présente invention concerne également l'utilisation de déchets agricoles provenant de céréales comme source d'aleurone.
Selon un mode de réalisation particulier, la présente invention concerne également l'utilisation de déchets agricoles provenant de céréales comme source d'aleurone,
lesdits déchets agricoles sont choisis parmi le son de toutes les graines de céréales parmi les différentes variétés de blé du genre *Triticum* (blé tendre, blé dur, blé de Khorasan...), l'épeautre, l'orge, l'avoine, le millet, le maïs, le riz, le seigle, le sarrasin, le quinoa, l'amarante, le sésame, le triticale ou un mélange de ces céréales.

La demande a également pour objet la valorisation des éléments à base de céréales, tels que les produits de mouture qui ne sont aujourd'hui pas utilisés.
En effet, le son de blé qui comprend le péricarpe, le tégument, la bande hyaline et la couche à aleurone, est éliminé lors de la mouture. Ainsi, l'aleurone qui reste attaché à la couche hyaline au cours du broyage est donc retiré avec les couches extérieures des grains.

L'aleurone peut être utilisée de manière fractionnée et non fractionnée.

L'aleurone peut être extraite par des procédés permettant le fractionnement de la couche à aleurone. L'aleurone est ainsi extraite, isolée et purifiée, et ces composants peuvent être réintroduits dans les produits alimentaires, médicaments et/ou compléments alimentaires sans modifier leurs propriétés.

Les variantes de réalisation pour l'utilisation de la couche à aleurone sont :
- sans fractionnement : en addition de certains aliments, de médicaments, de compléments nutritionnels ciblant la population générale et/ou des personnes vivant dans des zones contaminées et/ou des personnes potentiellement à risque (nourrissons et enfants consommant beaucoup de lait par exemple), d'aliments destinés à l'alimentation de Personnes Particulières et même en addition des aliments, médicaments et/ou compléments nutritionnels destinés aux animaux
- avec fractionnement ou tout autre procédé équivalent connu de l'homme de l'art : en addition de certains aliments, de médicaments, de compléments nutritionnels ciblant la population générale et/ou des personnes vivant dans des zones contaminées et/ou des personnes potentiellement à risque (nourrissons et enfants consommant beaucoup de lait par exemple), d'aliments destinés à l'alimentation de Personnes Particulières et même en addition des aliments, médicaments et/ou compléments nutritionnels destinés aux animaux

L'aleurone (seul, en association avec tout autre molécule ou aliment et ce, quelque soit le processus d'isolation) peut être utilisé dans les contextes suivants :
- en addition de certains aliments : les aliments à base de panure (poisson pané, toute autre viande panée, tourton...), les aliments de boulangerie (tout type de pain, pâte à pizza, pâte à tarte, gressini, farine, gâteau, crumble, préparation pour gâteaux...), dans les produits à base de céréales (céréales du matin pour accompagner le lait, chocolat aux céréales...), les produits laitiers (lait infantile, lait écrémé en poudre, lait concentré, les fromages enrobés, yaourts...), les aliments pour enfants (petits pots...), mais aussi la bière, les chips, la chapelure,...
   En effet, les aliments de boulangerie, les produits à base de céréales ou les panures du fait de leur composition sont de bons moyens d'administrer l'aleurone pour contrer les effets des PCBs contenus dans les autres aliments.
- en addition de certains médicaments, notamment pour pallier aux effets d'une contamination aiguë aux POPs comme les PCBs ou pour pallier à des effets toxiques similaires et/ou proches (maladies présentant des atteintes mitochondriales ou médicaments induisant des problèmes d'hépato et neuro toxicité)
- en addition de certains compléments nutritionnels, produits diététiques ou régimes amaigrissants (les PCBs étant stockés dans les graisses, un régime amaigrissant va entrainer leur mobilisation)
- en tant que ADAPP
- en tant qu'additifs alimentaires
- Dans les préparations des mélanges pour nutrition entérale, orale ou parentérale

Cette complémentation cible :
- la population générale du fait de la présence inévitable de ces polluants dans l'alimentation, y compris dans les produits issus de l'agriculture biologique,
- les personnes vivant dans des zones contaminées,
- les personnes potentiellement à risques (nourrissons et enfants consommant beaucoup de lait par exemple).

Généralement la couche à aleurone est éliminée dans les fractions des sons avec les enveloppes du grain. Outre l'effet bénéfique pour la santé, l'utilisation de l'aleurone pourrait constituer un enjeu de taille pour la valorisation des produits de mouture qui sont aujourd'hui inutilisés. Cet intérêt de la couche à aleurone pourrait être également utile pour valoriser les produits alimentaires à bases de céréales complètes.

### FIGURES

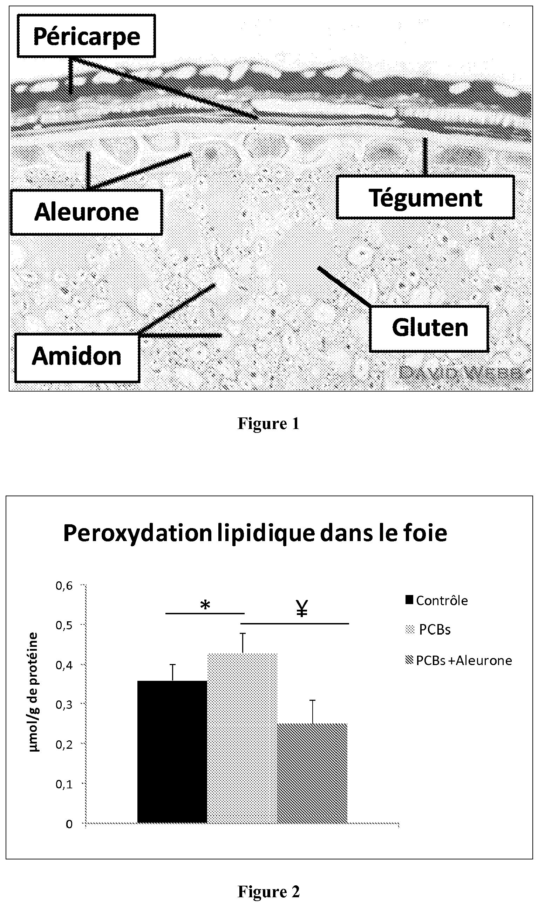

**Figure 1** : Elle représente la coupe histologique d'un grain de blé. En partant de l'extérieur vers l'intérieur, le grain de blé comprend le péricarpe, puis le tégument de la graine, puis la bande hyaline (non montrée), bande transparente sur laquelle s'étale l'assisse protéique, la couche à aleurone, formée de grosses structures protéiques empilées, les grains d'aleurone. Sous cette enveloppe, vient ensuite l'amande ou albumen farineux qui présente des grains d'amidon enchâssés dans un mince réseau de protéines quasi insolubles, le gluten.
**Figure 2** **:** Elle représente l'effet d'une administration de lait contaminé aux PCBs pendant 60 jours sur la peroxydation lipidique dans le foie. La colonne de gauche en noir représente le résultat sans PCBs (contrôle), celle du milieu en gris clair représente le résultat avec PCBs, et celle de droite en gris foncé représente le résultat avec PCBs et ingestion d'aleurone. L'axe des ordonnées représente la quantité de composés oxygénés réagissant avec l'acide thio-barbiturique ou « *Thiobarbituric acid reactive substances* » dans le foie en µmol de Malonedialdéhyde (MDA) /g de protéine, le MDA étant un produit issu de la peroxydation lipidique et qui interagit avec l'acide thiobarbiturique.

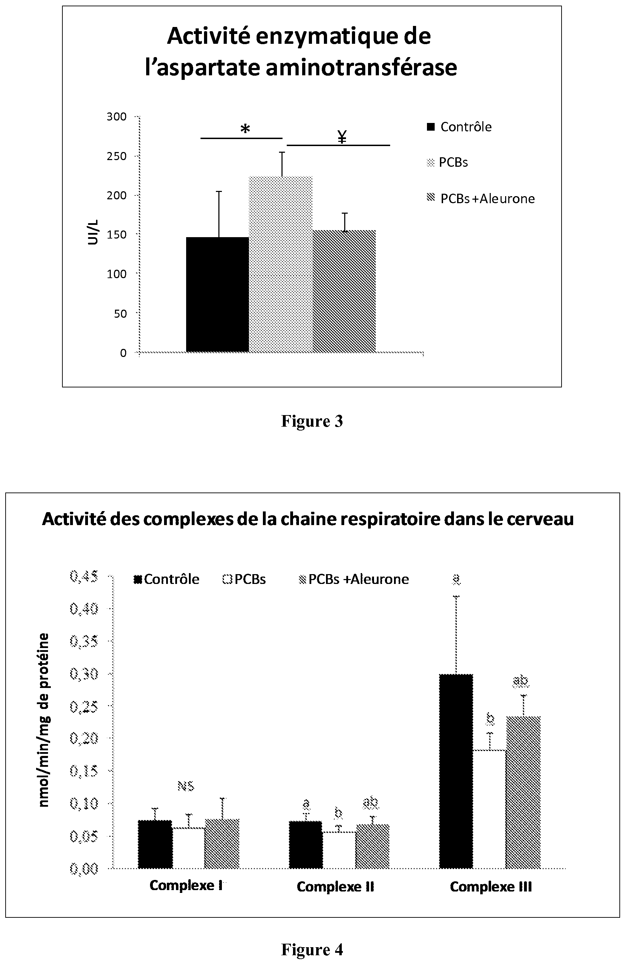

**Figure 3** : Elle représente l'effet d'une administration de lait contaminé aux PCBs pendant 60 jours sur l'activité de l'aspartate aminotransférase dans le foie. La colonne de gauche en noir représente le résultat sans PCBs (contrôle), celle du milieu en gris clair représente le résultat avec PCBs, et celle de droite en gris foncé représente le résultat avec PCBs et ingestion d'aleurone. L'axe des ordonnées représente l'activité enzymatique de l'aspartate aminotransférase, exprimée en UI/l.
**Figure 4** **:** Elle représente l'effet d'une administration de lait contaminé aux PCBs pendant 60 jours sur l'activité des complexes de la chaine respiratoire du cerveau.
   Chaque valeur représente la moyenne ± SD, n = 7 par groupe. a,b: les résultats annotés avec des lettres différentes sont significativement différents (p<0.05). NS: non significatif.
   Dans chaque triplet de colonnes, la colonne de gauche en noir correspond au résultat sans PCBs (contrôle), la colonne du milieu en blanc correspond au résultat avec PCBs, la colonne de gauche en gris correspond au résultat avec PCBs et ingestion d'aleurone.
   L'axe des ordonnées représente la disparition du substrat de la réaction en nmol/min/mg de protéine, à savoir du NADH pour le complexe I et du 2,6-dichloroindophénol (DCIP) pour le complexe II, et l'apparition du produit de la réaction en nmol/min/mg de protéine, à savoir du cytochrome réduit pour le complexe III.

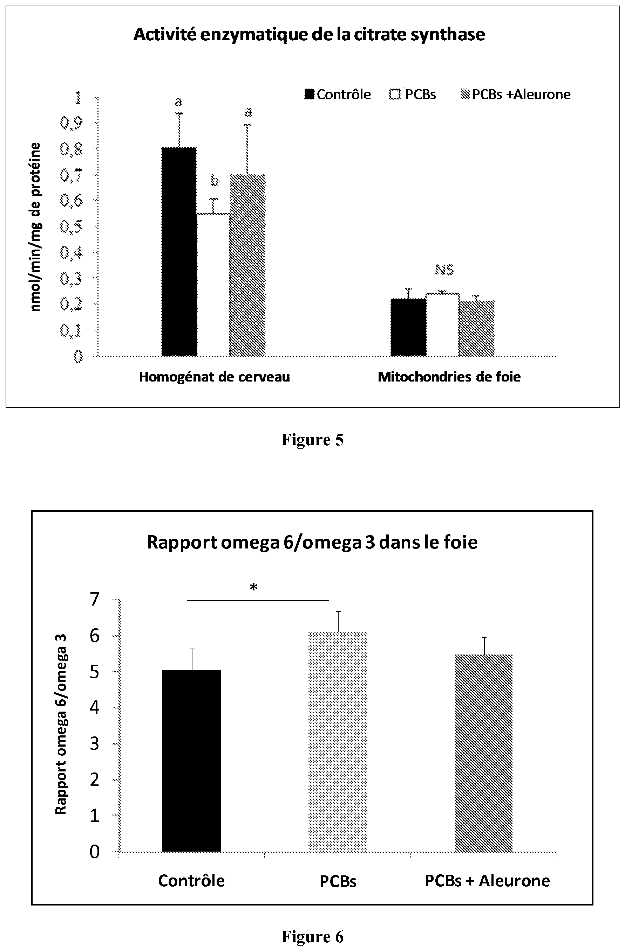

**Figure 5** : Elle représente l'effet d'une administration de lait contamine aux PCBs avec ou sans prise d'aleurone pendant 8 semaines sur l'activité de la citrate synthase. Chaque valeur représente la moyenne ± SD, n = 7 par groupe. a,b: les résultats annotés avec des lettres différentes sont significativement différents (p<0.05). NS: non significatif.
   Dans chaque triplet de colonnes, la colonne de gauche en noir correspond au résultat sans PCBs (contrôle), la colonne du milieu en blanc correspond au résultat avec PCBs, la colonne de droite en gris correspond au résultat avec PCBs et ingestion d'aleurone.
   Le triplet de colonnes de gauche présente l'activité de la citrate synthase dans le cerveau, le triplet de droite présente l'activité de la citrate synthase dans le foie.
   L'axe des ordonnées représente la production de trinitrobenzène (TNB) en nmol/min/mg de protéine.
**Figure 6** **:** Elle représente l'effet d'une administration de lait contaminé aux PCBs pendant 60 jours sur le rapport oméga 6/ oméga 3 des acides gras du foie. La colonne de gauche en noir représente le résultat sans PCBs (contrôle), celle du milieu en gris clair représente le résultat avec PCBs, et celle de droite en gris foncé représente le résultat avec PCBs et ingestion d'aleurone. L'axe des ordonnées représente le rapport oméga 6/ oméga 3.

### EXEMPLES

Les exemples 1 à 4 concernent la toxicité des PCBs pour le foie.

### Exemple 1

Cette étude nécessite deux laits : un lait témoin et un lait contaminé par les PCBs.

Le lait témoin est constitué à partir d'un pool de chèvres (8 chèvres) non exposées aux PCBs.

Le lait contaminé est préparé à partir du pool de chèvres utilisées pour constituer le lait témoins (8 chèvres), après une contamination par les PCBs. Les chèvres ont été contaminées par les PCBs par ingestion de sol du fait que les sols concentrent les polluants organiques et qu'une chèvre au pâturage ingère plus de 2% de sa ration alimentaire de sol par jour.

**Tableau 1 : Concentrations moyennes en PCB indicateurs (PCBs 28, 52, 101, 138, 118, 153, 180) retrouvées dans le lait témoin et le lait contaminé, ainsi que les concentrations moyennes totales e PCB-DL et PCB-NDL.**

| Molécules | Lait témoin ng/g de MG (Matière Grasse) | Lait contaminé ng/g de MG |
|---|---|---|
| PCB 28 | 0.23 | 3.42 |
| PCB 52 | 0.32 | 0.68 |
| PCB 101 | 0.78 | 1.99 |
| PCB 138 | 1.04 | 18.4 |
| PCB 118 | 1.10 | 11.1 |
| PCB 153 | 1.73 | 27.5 |
| PCB 180 | 0.63 | 33.2 |
| PCB-DL totaux | 1.72 | 2.07 |
| PCB-NDL totaux | 4.74 | 85.2 |

Les laits (témoins et contaminés) étant issus des mêmes chèvres, leurs compositions biochimiques (en protéine et en matière grasse) sont supposées être proches. Les rats ont été gavés dans un cas avec du lait témoin, et dans l'autre avec du lait contaminé, les volumes de lait contaminé et de lait témoin administrés étant identiques.

Deux pools de 10 rats ont été utilisés : un pool (n=10) correspondant aux rats contrôles, un pool correspondant aux rats exposés aux PCBs.

Les rats exposés aux PCBs ont reçu des doses de 23.3 ng de PCBs- i /kg PC (PCBs indicateurs / kg Poids Corporel) contre seulement 1.4 ng/kg PC chez les rats contrôles.

Cette dose est deux fois supérieure à la dose journalière admissible, mais reste en dessous des doses habituellement utilisées lors des travaux de recherche toxicologique menés in vivo (de l'ordre du µg/kg PC).

La dose utilisée ici équivaut à une exposition alimentaire réaliste aux PCBs. Elle présente également l'avantage de contenir d'autres familles de composés comme les PCDD/Fs (les dioxines PCDD-Fs regroupent les polychlorobenzodioxines : PCDD, et les polychlorodibenzofuranes : PCDF) qui présentent également un potentiel toxique important (les données sur la teneur des PCDD/Fs dans le lait sont non connues).

L'activité des transaminases plasmatiques du foie est mesurée (Figure 3 et Tableau 3) à l'aide d'un kit commercial (Roche) permettant de suivre l'oxydation du NADH à 340 nm qui est directement proportionnelle à l'activité catalytique de l'aspartate aminotransférase et de l'alanine aminotransférase.

L'exposition aux PCBs chez les rats provoque une augmentation de l'activité de l'aspartate aminotransférase du foie et de l'alanine aminotransférase du foie, d'environ 30%.

L'aspartate aminotransférase et l'alanine aminotransférase se trouvent en grande quantité dans le foie. Une atteinte du foie entraine la libération de ces enzymes dans le sang et l'augmentation de leurs taux dans le plasma sanguin.

Une augmentation de l'activité de l'aspartate aminotransférase et de l'alanine aminotransférase plasmatique traduit une destruction progressive des cellules du foie, et donc une intoxication.

### Exemple 2

Les rats ont été exposés aux PCBs selon le protocole décrit dans l'exemple 1.

La consommation mitochondriale d'oxygène hépatique est mesurée pour les complexes respiratoires I, II (Tableau 2).

La consommation d'oxygène des mitochondries isolées en nmol O₂/ml est mesurée dans un oxygraphe (Hansatech Instrument).

La consommation d'oxygène par les mitochondries est mesurée à 30°C dans un milieu de respiration tamponné à pH 7.2 (125 mM KCl, 20 mM Tris Base, 1 mM EGTA) auquel est ajouté 0.15% de BSA délipidée (FFA) et 5 mM Pi Tris pH 7.2. Selon le substrat utilisé, 0.5 à 1 mg/ml de suspension mitochondriale sont placés dans la chambre d'oxygraphie. Après addition des substrats, l'état 2 de la respiration est mesuré. L'adjonction de 300 µM d'ADP permet d'obtenir la respiration à l'état 3, c'est-à-dire en condition phosphorylante. La respiration oligomycine-insensible est obtenue après addition de 1.25 µg/ml d'oligomycine, un inhibiteur spécifique de la rentrée des protons dans la matrice via l'ATP synthase. Cette respiration, dite à l'état 4, est le reflet d'une rentrée passive des protons à travers la membrane interne mitochondriale.

Les mitochondries sont énergisées par différents substrats, soit du Glutamate/Malate (5 mM/2.5 mM) qui permet d'étudier la chaîne respiratoire à partir du complexe I (NADH-ubiquinone déshydrogénase), soit du succinate (5 mM) additionné de 2.5 µM de roténone, afin d'étudier la chaîne respiratoire à partir du complexe II (succinate déshydrogénase).

**Tableau 2 : Effet d'une faible dose de PCBs provenant de lait contaminé aux PCBs avec ou sans prise d'aleurone sur la consommation mitochondriale d'oxygène dans les mitochondries hépatiques.**

| Respiration (nmol O₂.min⁻¹.mg prot⁻¹) | | | | | | |
|---|---|---|---|---|---|---|
| | Glutamate/Malate (Complexe I) | | | Succinate (Complexe II) | | |
| | Etat 3 | Etat 4 | RCR | Etat 3 | Etat 4 | RCR |
| Contrôle | 76.9 ±6.36^{a} | 11.25 ±1.98^{a} | 7.00 ±1.18^{a} | 111.3 ±10.7^{a} | 17.9 ±3.68^{a} | 6.55 ±2.05^{a} |
| PCBs | **60.7 ±9.69^{b}** | 10.55 ±2.7^{Ab} | 6.05 ±1.52^{a} | **79.3 ±14.2^{b}** | **13.4 ±4.86^{b}** | 6.21 ±1.12^{a} |
| PCBs +Aleurone | **60.6 ±11.8^{a}** | **14.34 ±5.22^{b}** | **4.49 ±0.94^{b}** | **87.6 ±12.4^{b}** | 20.3 ±4.09^{a} | **4.39 ±0.59^{b}** |

Chaque valeur représente la moyenne ± SD, n=10 par groupe. a,b les résultats annotés avec des lettres différentes sont significativement différents (p<0.01). Aleurone : granulés d'aleurone de blé. Le taux de consommation d'oxygène est mesuré en présence soit de 5 mM de glutamate et 2.5 mM de malate, ou soit 5 mM de succinate. L'état 3 et 4 ont été mesuré après l'addition de 500 µM d'adénosine diphosphate (ADP) et 0.50 µg d'oligomycine/mg de protéine, respectivement. Le rapport de contrôle respiratoire (RCR) est le rapport état 3/ état 4.

Les PCBs réduisent significativement la respiration des mitochondries dans le foie en condition phosphorylante, et ce, quel que soit le substrat de respiration (réduction de 21% à 30%).

### Exemple 3

Les rats ont été exposés aux PCBs selon le protocole décrit dans l'exemple 1.

La peroxydation lipidique a été mesurée dans le foie par l'intermédiaire de la mesure des substances réactives avec l'acide thiobarbiturique (TBARs) (en µmol/g de protéine) (Figure 2). Cette mesure repose sur la formation en milieu acide et à chaud entre le malondialdéhyde et deux molécules d'acide thiobarbiturique (TBA), d'un pigment absorbant à 532 nm, extractible par les solvants organiques comme le butanol (Richard et al., 1992). Pour cela, 100 µL d'échantillon sont ajoutés à 750 µL de solution contenant de l'acide thiobarbiturique (TBA) 0,55 mM à pH 7,4 et de l'acide perchlorique à 7%. Le mélange obtenu est chauffé à 95°C au bain-marie pendant 1h, provoquant une hydrolyse du malondialdéhyde (MDA). Il se forme alors un complexe MDA-TBA2 qui est extrait par le butanol pendant 2 minutes. Après centrifugation, le complexe est quantifié par fluorimétrie à 532 nm. La courbe de calibration est obtenue avec une gamme de solution de 1,1,3,3-TétraEthoxyPropane (TEP) à 20 mmol/L, comprise entre 1 et 8 mmol/L.

La quantité de TBARs dans le foie, suite à l'exposition aux PCBs, augmente de 20%.

Les TBARs traduisent la peroxydation des lipides, suite à un stress oxydatif. Cette expérience montre que l'exposition de rats aux PCBs entraine une augmentation des lésions cellulaires dans le foie.

### Exemple 4

Les rats ont été exposés aux PCBs selon le protocole décrit dans l'exemple 1.

Le rapport oméga 6/oméga 3 hépatique a été mesuré chez les rats contrôles et les rats exposés aux PCBs (Figure 6).

Le rapport oméga6/oméga3 pour les rats non exposés aux PCBs est égal à 5.06, contre 6,11 pour les rats exposés aux PCBs.

L'exposition aux PCBs entraine une augmentation du rapport oméga6/oméga 3 de 20%, et témoigne donc d'une modification de la synthèse des acides gras polyinsaturés.

Les exemples 5 et 6 concernent de la toxicité des PCBs sur le cerveau.

### Exemple 5

Les rats ont été exposés aux PCBs selon le protocole décrit dans l'exemple 1. L'activité des complexes I, II, III des mitochondries du cerveau a été mesurée (Figure 4).

L'activité du complexe I a été mesuré par le biais d'un dosage consistant à mesurer par spectrophotométrie l'oxydation du NADH, catalysée par le complexe I, à λ = 340 nm en présence d'un analogue de l'ubiquinone, le décylubiquinone. En effet, la diminution de l'absorbance est proportionnelle à l'activité du complexe I. De la roténone, un inhibiteur spécifique du complexe I, est ensuite ajoutée pendant 2 minutes afin de mesurer l'oxydation non spécifique du NADH. L'activité du complexe II a été mesurée en suivant la réduction du 2,6-dichloroindophénol (DCIP), un composé de couleur bleue absorbant la lumière à λ = 600 nm, la diminution de l'absorbance étant proportionnelle à l'activité du complexe II. Lors de la mesure par spectrophotométrie, de la roténone et de l'Antimycine A (inhibiteur spécifique du complexe III) ont été utilisés afin de ne mesurer que l'activité spécifique au complexe II.

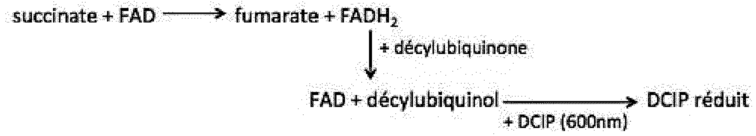

L'activité du complexe III a été mesurée par un dosage basé sur la mesure spectrophotométrique à λ = 550 nm de la réduction du cytochrome c en présence de décylubiquinone. La réaction est réalisée en présence puis en absence d'Antimycine A pour obtenir l'activité spécifique du complexe III. L'augmentation de l'absorbance est alors proportionnelle à l'activité du complexe III.

Les résultats montrent :
- l'absence d'une différence significative, entre l'activité du complexe I entre les rats exposés aux PCBs et celles des rats non-exposés aux PCBs.
- une diminution de l'activité du complexe II chez les rats exposés aux PCBs de 24% par rapport à celle des rats non-exposés aux PCBs.
- une diminution de l'activité du complexe III chez les rats exposés aux PCBs de 39% par rapport à celle des rats non-exposés aux PCBs.

La contamination des rats par les PCBs provoquent donc une diminution de l'activité des complexes de la chaine respiratoire dans le cerveau.

### Exemple 6

Les rats ont été exposés aux PCBs selon le protocole décrit dans l'exemple 1.

La mesure de l'activité enzymatique de la citrate synthase dans le cerveau, a été réalisée (Figure 5).

La citrate synthase est la première enzyme du cycle de l'acide citrique (cycle de Krebs) située dans la matrice mitochondriale. Le dosage de l'activité de la citrate synthase est réalisé en suivant la réaction de l'acide 5,5'-dithio-bis(2-nitrobenzoïque) ou DTNB avec les groupements thiols (-SH) du Coenzyme A-SH (CoA-SH) pour former du trinitrobenzène (TNB), un composé jaune absorbant à λ = 412 nm dont l'apparition est suivie par spectrophotométrie.

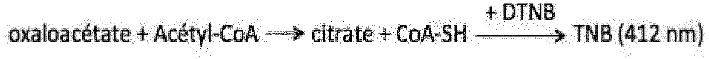

Les résultats montrent une diminution de 30% de l'activité de l'activité de la citrate synthase dans le cerveau chez des rats exposés aux PCBs.

La citrate synthase est une enzyme impliquée dans le cycle de Krebs, et la baisse de son activité témoigne d'une altération du métabolisme cellulaire.

Les exemples qui suivent concernent l'effet de l'aleurone dans la prévention de la toxicité hépatique et cérébrale induites par les PCBs.

### Exemple 7

Cette étude nécessite deux lait : un lait témoin et un lait contaminé par les PCBs. Ces laits ont été préparés selon le protocole décrit à l'exemple 1 et les rats ont été gavés avec ces laits comme indiqué dans l'exemple 1.

Afin de faciliter l'ingestion de l'aleurone par les rats, des boulettes ont été constituées d'une part de poudre d'aleurone (2g pour un apport énergétique de 72.5 Kcal/j), et d'autre part de sucre blanc commercial (0.25g) pour augmenter l'appétence des boulettes. Des boulettes témoins ont été également préparées et distribuées aux rats ; elles contenaient de l'aliment standard A04 (2g) et du sucre blanc en poudre (0.25g).

Trois groupes de rats (n=10) ont été utilisés. Les rats ont été exposés à des doses de 23.3 ng de PCBs-i/kg PC (contre seulement 1.4 ng/kg PC chez les rats contrôles).

Les rats sont donc ici répartis en trois groupes :
- groupe 1 : les rats sont gavés avec du lait non contaminé, et nourris avec l'aliment standard A04 et des boulettes témoins sans aleurone (contrôle)
- groupe 2 : les rats sont gavés avec du lait contaminé par des PCBs, et nourris avec l'aliment standard A04 et des boulettes témoins sans aleurone (PCBs)
- groupe 3 : les rats sont gavés avec du lait contaminé par des PCBs, et nourris avec l'aliment standard A04 et des boulettes avec aleurone. (PCBs+Aleurone)

La peroxydation lipidique (Figure 2) a été mesurée dans le foie.

Les résultats montrent l'augmentation de la quantité de TBARs dans le foie, chez les rats exposés aux PCBs, en comparaison des rats témoins. D'autre part, la comparaison de la quantité de TBARs dans le foie, entre des rats exposés aux PCBs, et des rats exposés aux PCBs mais ayant ingéré de l'aleurone, montre une diminution de la quantité de TBARs de 58%.

La consommation d'aleurone permet de diminuer la quantité de TBARs dans le foie, chez les rats exposés aux PCBs.

### Exemple 8

Les rats ont été exposés aux PCBs selon le protocole décrit dans l'exemple 7.

L'activité de l'aspartate aminotransférase plasmatique est mesurée chez les rats témoins, les rats exposés aux PCBs, les rats exposés aux PCBs et ayant ingéré de l'aleurone (Figure 3).

L'activité de l'aspartate aminotransférase augmente chez les rats exposés aux PCBs en comparaison de celle des rats témoins. Cependant, on constate une diminution de l'activité de l'aspartate aminotransférase chez les rats exposés aux PCBs et ayant ingéré de l'aleurone en comparaison de celle de rats ayant uniquement été exposés aux PCBs.

L'ingestion d'aleurone par les rats exposés aux PCBs entraine une diminution de l'activité de l'aspartate aminotransférase.

Le tableau 3 ci-après résume les résultats obtenus et figurant dans les exemples 7 et 8.

**Tableau 3 : Effets de faibles doses de PCBs provenant de lait contamine aux PCBs avec ou sans prise d'aleurone sur les paramètres de stress oxydant hépatique et les transaminases dans le plasma.**

| | Contrôle | PCBs | PCBs + Aleurone |
|---|---|---|---|
| *Paramètres mesurés dans le plasma* | | | |
| TBAR's, µmol/g protéine | 0.046 ±0.006^{a} | **0.054 ±0.004^{b}** | 0.045 ±0.005^{a} |
| SH groupements, µmol/g protéine | 4.7 ±0.37 | 4.2 ±0.23 | 4.8 ±0.46 |
| FRAP, µmol/g protéine | 4.5 ±0.76 | 5.2 ±0.71 | 4.6 ±0.98 |
| GPx, UI/g protéine | 111.3 ±12.5 | 97.1 ±13.5 | 108.1 ±7.1 |
| Dommages oxydatifs de l'ADN (comet)² | 5.19 ±1.63 | 5.62 ±1.87 | 5.95 ±2.53 |
| GST, UI/g protéine | 1675.3 ±85.6^{a} | **1557.2 ±93.0^{b}** | **1507.9 ±96.2^{b}** |
| ASAT, UI/L | 146.4 ±41.2^{a} | **220.9 ±32.2^{b}** | 154.7 ±19.0^{a} |
| ALAT, UI/L | 46.3 ±8.5^{a} | **60.2 ±9.8^{b}** | 51.0±7.3^{ab} |
| | | | |

| *Paramètres mesurés dans le foie* | | | |
|---|---|---|---|
| TBARs, µmol/g protéine | 0.36 ±0.04^{a} | **0.43 ±0.05^{c}** | **0.25 ±0.06^{b}** |
| SH groupes, µmol/g protéine | 66.2 ±8.8 | 66.2 ±7.4 | 65.0 ±8.9 |
| FRAP, µmol/ g protéine | 92.3 ±23.6 | 93.2 ±16.9 | 107 ±4.0 |
| GPx, UI/g protéine | 1364.4 ±157.4 | 1279.8 ±287.2 | 1382.9 ±241.5 |

Les valeurs représentent les moyennes ± SD, n=8 par groupe. a.b.c : les résultats annotés avec des lettres différentes sont significativement différents (p<0.05). TBARs : substances réactives avec l'acide thiobarbiturique, groupements SH: groupements Thiols, FRAP : réducteur de capacité ferrique, GPx: glutathion peroxydase, ASAT: Aspartate aminotransférase, ALAT: Alanine aminotransférase. ² Unités arbitraires.

### Exemple 9

Les rats ont été exposés aux PCBs, selon le protocole décrit dans l'exemple 7.

Le rapport oméga 6/oméga 3 hépatique a été mesuré chez les rats contrôles et les rats exposés aux PCBs, les rats exposés aux PCBs et ayant ingéré de l'aleurone (Figure 6).

La prise d'aleurone dans le cas d'ingestion de PCBs permet d'éviter une augmentation du rapport oméga 6/oméga3.

Le tableau 4 ci-après résume les résultats obtenus.

**Tableau 4 : Effet d'une administration pendant 8 semaines de lait contaminé aux PCBs avec ou sans prise d'aleurone sur les taux d'acides gras dans le foie chez le rat.**

| | Contrôle | | | PCB | | | PCB + Aleurone | | |
|---|---|---|---|---|---|---|---|---|---|
| | Moyenne | SD | | Moyenne | SD | | Moyenne | SD | |
| Acides gras (% du total des acides gras) | | | | | | | | | |
| C14:0 | **0.60** | 0.09 | A | **0.46** | 0.10 | AB | **0.22** | 0.18 | B |
| C16:0 | **23.84** | 1.39 | A | **22.33** | 2.30 | AB | **19.01** | 2.89 | B |
| C16:1n-7 | **3.61** | 0.84 | | **3.51** | 1.64 | | **1.85** | 0.85 | |
| C18:0 | **14.81** | 0.84 | | **14.77** | 0.65 | | **15.02** | 1.18 | |
| C18:1n-9 | **7.59** | 0.87 | | **7.97** | 0.81 | | **7.38** | 0.66 | |
| C18:1n-7 | **5.09** | 0.65 | A | **4.46** | 0.67 | A | **4.29** | 1.33 | B |
| C18:2n-6 | **15.96** | 1.03 | | **16.51** | 2.03 | | **18.02** | 1.79 | |
| C18:3n-6 | **0.19** | 0.03 | | **0.19** | 0.09 | | **0.26** | 0.04 | |
| C20:0 | **0.05** | 0.01 | A | **0.01** | 0.01 | B | **0.04** | 0.01 | A |
| C18:3n3 (a-Linoléique) | **0.27** | 0.06 | | **0.26** | 0.14 | | **0.32** | 0.11 | |
| C18:4n3 | **0.00** | 0.01 | B | **0.00** | 0.00 | B | **0.03** | 0.01 | A |
| C20:2n-6 | **0.31** | 0.06 | | **0.25** | 0.06 | | **0.31** | 0.07 | |
| C20:3n-9 | **0.18** | 0.04 | | **0.17** | 0.05 | | **0.14** | 0.03 | |
| C20:3n-6 | **1.14** | 0.12 | | **1.05** | 0.28 | | **1.01** | 0.33 | |
| C20:4n-6 | **19.18** | 1.23 | B | **21.42** | 1.97 | AB | **23.89** | 2.93 | A |
| C20:5n-3 (EPA) | **0.57** | 0.07 | | **0.66** | 0.17 | | **0.65** | 0.19 | |
| C22:4n-6 | **0.35** | 0.03 | B | **0.31** | 0.04 | B | **0.47** | 0.09 | A |
| C22:5n-3 | **0.83** | 0.09 | AB | **0.71** | 0.09 | B | **1.02** | 0.16 | A |
| C22:6n-3 (DHA) | **5.57** | 0.86 | A | **4.64** | 0.63 | B | **6.06** | 0.72 | A |
| TOTAL | 100 | | | 100 | | | 100 | | |
| n=3 | 7.80 | | A | 6.66 | | B | 8.08 | | A |
| n=6 | 38.77 | | B | 40.31 | | B | 43.96 | | A |
| ratio | 5.06 | | B | 6.11 | | A | 5.48 | | B |

Les valeurs représentent les moyennes ± SD, n=10 par groupe. a.b.c : les résultats annotés avec des lettres différentes sont significativement différents (p>0.05) one-way ANOVA (analyse de variance à un facteur).

### Exemple 10

L'activité des complexes I, II, III de la chaine respiratoire du cerveau a été mesurée chez des rats témoins, des rats exposés aux PCBs et les rats exposés aux PCBs ayant ingéré de l'aleurone (Figure 4).

Il n'y a pas de différence significative dans la consommation d'oxygène, entre des rats témoins, des rats exposés aux PCBs et des rats exposés aux PCBs mais ayant ingéré de l'aleurone, dans le cas du complexe 1.

L'exposition aux PCBs entraine une diminution de l'activité du complexe II, et l'ingestion d'aleurone par les rats exposés aux PCBs permet une augmentation de l'activité du complexe II.

L'exposition aux PCBs entraine une diminution de l'activité du complexe III, et l'ingestion d'aleurone par les rats exposés aux PCBs permet une augmentation de l'activité du complexe III. L'ingestion d'aleurone permet donc d'augmenter l'activité des complexes II et III de la chaine respiratoire du cerveau, chez les rats exposés aux PCBs.

De plus, le tableau 5 renseigne sur les paramètres de stress oxydant dans le cerveau.

**Tableau 5 : Effets d'une faible dose de PCBs provenant de lait contaminé aux PCBs avec ou sans prise d'aleurone sur les paramètres de stress oxydant dans le cerveau.**

| | Contrôle | PCBs | PCBs + Aleurone |
|---|---|---|---|
| *Paramètres* | | | |
| TBARs, µmol/g protéine | 0.45 ±0.12^{a} | **0.56 ±0.09^{b}** | 0.48 ±0.07^{a} |
| SHgroupes,µmol/g protéine | 96.9 ±4.2 | 96.2 ±4.6 | 95.5 ±1.9 |
| FRAP, µmol/g protéine | 149.9 ±31.9 | 158.5 ±20.7 | 149.9 ±28.5 |
| GPx, UI/g protéine | 171.6 ±11.4 | 172.3 ±9.2 | 175.2 ±18.7 |

Les valeurs représentent les moyennes ± SD, n=7 par groupe. a.b : les résultats annotés avec des lettres différentes sont significativement différents (p<0.05). TBARs : substances réactives avec l'acide thiobarbiturique, groupements SH: groupements Thiols, FRAP: réducteur de capacité ferrique, GPx : glutathione peroxydase.

### Exemple 11

L'activité de la citrate synthase a été mesurée chez des rats témoins, des rats exposés aux PCBs, et des rats exposés aux PCBs et ayant ingéré de l'aleurone (Figure 5).

Dans le cerveau, l'exposition aux PCBs entraine une diminution de l'activité de la citrate synthase chez des rats exposés aux PCBs, par rapport à celle des rats témoins, et l'ingestion d'aleurone par les rats exposés aux PCBs entraine une augmentation de l'activité de la citrate synthase, en comparaison de celle de rats uniquement exposés aux PCBs.

Dans le foie, il n'y a pas de différence significative entre l'activité de la citrate synthase des rats témoins, des rats exposés aux PCBs et des rats exposés aux PCBs ayant ingéré de l'aleurone.

### Exemple 12

Mesure du niveau de PCBs dans les tissus des animaux recevant des PCBs et de l'aleurone afin de le comparer avec le niveau de PCBs dans les tissus des animaux recevant uniquement des PCBs. Le but est de vérifier l'absence de diminution de l'accumulation des PCBs dans les tissus hépatique et adipeux ainsi que dans le cerveau en présence d'aleurone.

On peut voir, dans le tableau 6, que dans le tissu adipeux (tissu préférentiel de l'accumulation de PCBs dans l'organisme) et dans le foie, il n'y a pas de diminution de la concentration de PCBs dans le groupe d'animaux recevant PCBs et aleurone par rapport au groupe d'animaux recevant uniquement des PCBs. On observe également que les PCBs ne s'accumulent pas dans le cerveau. L'aleurone n'a donc aucun effet sur l'absorption des PCBs dans les tissus.

A noter une augmentation de la concentration de certains PCBs dans le groupe recevant l'aleurone en plus des PCBs. Ceci s'explique par le fait que l'aleurone utilisé n'est pas exempte de polluants.

Le tableau 6 ci-après résume les résultats obtenus.

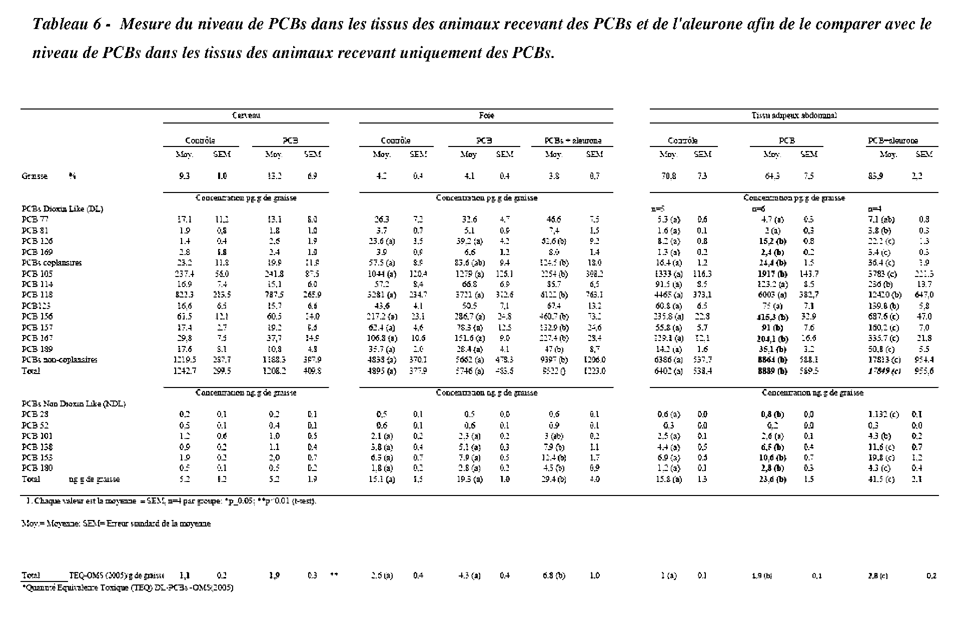

### Discussion :

Les résultats montrent que même apportés à des doses faibles (doses réalistes représentatives de l'exposition à laquelle un individu est quotidiennement soumis), les PCBs augmentent la peroxydation lipidique (TBARs) de 20% dans le foie et le cerveau et d'environ 30% les transaminases du plasma (Tableau 3).

De plus, ils perturbent de manière significative la fonction mitochondriale dans le foie (Tableau 2) mais également l'activité de certains complexes de la chaine respiratoire du cerveau (Figure 4). Ils réduisent significativement la respiration des mitochondries du foie de rat en conditions phosphorylantes (Tableau 2) et ce, quel que soit le substrat de respiration (de 21 à 30%) (Tableau 2). De même, ils réduisent significativement l'activité des complexes II et III du cerveau (de respectivement 24% et 39%) (Figure 4).

L'activité de la citrate synthase, une enzyme mitochondriale impliquée dans le cycle de Krebs est également altérée par les PCBs (diminution d'activité de 30%) dans le cerveau (Figure 5).

Par ailleurs, l'exposition réaliste aux PCBs entraine une augmentation du rapport omega6/omega3 de 20% due en particulier à une baisse du C22:6n-3 (Acide DocosaHexaenoique, DHA) de 17% (Tableau 4). Une augmentation de ce rapport est décrit dans la littérature comme défavorable (entrainant entre autres effets, une augmentation du risque cardiovasculaire, du risque de cancer, du risque d'obésité et de troubles métaboliques) (De Lorgeril M et al., 2012., Muhlhausler BS et al., 2013) (Tableaux 3 et 5).

Une alimentation enrichie en aleurone est capable de prévenir l'augmentation de la peroxydation lipidique au niveau du foie et du cerveau (Tableaux 3 et 5), de limiter l'augmentation des transaminases plasmatiques (Tableau 3) et, de façon partielle, les altérations de la chaine respiratoire (Tableau 1 et Figure 4) et de la citrate synthase (Figure 5) induites par les PCBs. Elle permet également de prévenir totalement l'augmentation du rapport omega6/omega3 induite par les PCBs (Tableaux 3 et 5).

Une alimentation enrichie en aleurone n'a aucun effet sur l'absorption des PCBs dans les tissus (Tableau 6). Ce mécanisme ne peut donc pas être à l'origine des effets sur la toxicité hépatique et cérébrale induite par un mélange de polluants observés dans le cadre de l'invention.

En conclusion, une alimentation enrichie avec de la couche à aleurone peut prévenir la toxicité hépatique et cérébrale induite par un mélange de polluants organiques persistants comme les PCBs.

## Revendications

1. Composition comprenant tout ou partie de la couche d'aleurone d'une céréale pour son utilisation dans la prévention ou le traitement de la toxicité hépatique et cérébrale induite par au moins un polluant, notamment contenu dans un aliment,
ledit polluant étant un PCB ou un mélange de PCBs et/ou un pesticide choisi parmi les organochlorés, les organophosphorés, les pyréthroïdes, les carbamates et les triazines,
la quantité d'aleurone administrée étant comprise de 0.01 g/kg de poids corporel (PC) à 0.5 g/kg PC, ou de 0.5 g / kg PC à 5 g / kg PC,
ou de 0.01 g/kg PC à 0.05 g/kg PC, ou de 0.05 g/kg PC à 0.1 g/kg PC, ou de 0.1 g/kg PC à 0.5 g/kg PC, ou de 0.5 g/kg PC à 1 g/kg PC, ou de 1 g/kg PC à 3 g/kg PC, ou de 3 g/kg PC à 6 g/kg PC.

2. Composition comprenant tout ou partie de la couche d'aleurone d'une céréale pour son utilisation selon la revendication 1, **caractérisée en ce que** ledit polluant est choisi parmi un PCB ou un mélange de PCBs appartenant au groupe des PCBs « dioxin-like », en particulier choisi parmi le PCB 126 (3,3',4,4',5-pentachlorobiphenyl), le PCB 118 (2,3,4,4',5-pentachlorobiphenyl), et/ou des PCBs « non dioxin-like » en particulier choisi parmi le PCB 28 (2,4,4'-Trichlorobiphenyl), le PCB 52 (2,2',5,5'-tetrachloro-1,1'-Biphenyl), le PCB 101 (2,2',4,5,5'-Pentachlorobiphenyl), le PCB 138 (2,2',3,4,4',5'-hexachlorobiphenyl), le PCB 153 (2,3',4,4',5-Pentachlorobiphenyl), le PCB 180 (2,2',3,4,4',5,5'-heptachlorobiphenyl), ou des dioxines ou des pesticides.

3. Composition comprenant tout ou partie de la couche d'aleurone d'une céréale pour son utilisation selon la revendication 1, **caractérisée en ce que** ladite utilisation est réalisée chez des sujets humains ou animaux dont les tissus contiennent des PCBs, notamment chez des sujets humains dont les tissus adipeux contiennent des PCBs à une dose allant de 14 à 1700 ng/g

4. Composition comprenant tout ou partie de la couche d'aleurone d'une céréale pour son utilisation selon la revendication 3, **caractérisée en ce que** ladite utilisation est réalisée chez des sujets humains dont le sérum contient des PCBs à une dose allant de 0.9 à 1.5 ng/ml, notamment de 5.4 à 9 ng/ml dans des situations d'exposition particulière et notamment de 900 à 1500 ng/ml lors d'une exposition professionnelle.

5. Composition comprenant tout ou partie de la couche d'aleurone d'une céréale pour son utilisation selon la revendication 4, **caractérisée en ce que** ladite utilisation est réalisée chez des sujets humains dont le lait maternel contient des PCBs à une dose allant de 238 à 271 ng/g et notamment 1090 ng/g dans des situations d'exposition particulière.

6. Composition comprenant tout ou partie de la couche d'aleurone d'une céréale pour son utilisation selon l'une des revendications 1 ou 2, **caractérisée en ce que** le polluant est administré à des doses allant de 3 ng/kg PC/jour à 6 mg/kg PC/jour,
notamment de façon chronique, notamment quotidiennement et en particulier à des doses allant de 3 à 50 ng/kg PC/jour, et notamment de 10 à 40 ng/kg PC/jour, plus particulièrement de 20 à 30 ng/kg/jour,
ou notamment de façon aiguë, à des doses supérieures à 1 µg/kg PC/jour, notamment de 1 mg/kg PC/jour à 3 000 mg/kg PC/jour, plus particulièrement de 500 µg/kg PC/jour à 6 mg/kg PC/jour.

7. Composition comprenant tout ou partie de la couche d'aleurone d'une céréale pour son utilisation selon l'une des revendications 1 à 6, **caractérisée en ce que** ladite céréale étant choisie parmi les différentes variétés de blé du genre *Triticum* (blé tendre, blé dur, blé de Khorasan...), l'épeautre, l'orge, l'avoine, le millet, le maïs, le riz, le seigle, le sarrasin, le quinoa, l'amarante, le sésame, le triticale, ladite céréale étant en particulier le blé, ou un mélange de ces céréales.

8. Composition comprenant tout ou partie de la couche d'aleurone d'une céréale pour son utilisation selon l'une des revendications 1 à 7, **caractérisée en ce que** ladite prévention ou traitement a lieu chez l'homme ou chez les animaux, notamment les animaux de compagnie ou de rente.

9. Composition comprenant tout ou partie de la couche d'aleurone d'une céréale pour son utilisation selon l'une des revendications 1 à 8, **caractérisée en ce que** la toxicité hépatique et cérébrale est provoquées par un stress oxydant, ou la toxicité hépatique et cérébrale est provoquée par un mécanisme non oxydant, ou la toxicité hépatique et cérébrale est provoquées exclusivement par un mécanisme non oxydant, ou la toxicité hépatique et cérébrale est provoquées non exclusivement par un mécanisme non oxydant, ou la toxicité hépatique et cérébrale est provoquées par un stress oxydant et/ou un mécanisme non oxydant.

10. Composition comprenant tout ou partie de la couche d'aleurone d'une céréale pour son utilisation selon l'une des revendications 1 à 9, **caractérisée en ce que** la toxicité hépatique et cérébrale est provoquées exclusivement par un mécanisme non oxydant, la toxicité hépatique et cérébrale étant l'augmentation du rapport oméga 6 sur oméga 3 en comparaison du rapport oméga 6 sur oméga 3 égal à 5.

11. Composition comprenant tout ou partie de la couche d'aleurone d'une céréale pour son utilisation selon l'une des revendications 1 à 9, **caractérisée en ce que** la toxicité hépatique et cérébrale est provoquées par un stress oxydant, la toxicité hépatique et cérébrale étant l'augmentation de la peroxydation lipidique dans le foie et le cerveau.

12. Composition comprenant tout ou partie de la couche d'aleurone d'une céréale pour son utilisation selon l'une des revendications 1 à 9, **caractérisée en ce que** la toxicité hépatique et cérébrale est provoquées par un stress oxydant et/ou un mécanisme non oxydant, la toxicité hépatique et cérébrale étant la diminution de l'activité de la citrate synthase, et/ou la perturbation de la chaine respiratoire mitochondriale dans le foie, et/ou la perturbation de l'activité des complexes de la chaine respiratoire mitochondriale dans le cerveau, et/ou l'augmentation de l'activité des transaminases dans le plasma.

13. Composition comprenant tout ou partie de la couche d'aleurone d'une céréale pour son utilisation selon l'une des revendications 1 à 9, **caractérisée en ce que** la dose unitaire d'aleurone de ladite composition étant comprise de 0.7 g à 200 g, en particulier 0.7 g à 100 g ou comprise 0.6 g à 180 g.

14. Composition alimentaire ou nutraceutique pour son utilisation pour inhiber la toxicité hépatique et cérébrale due aux polluants, ladite composition comprenant une quantité d'aleurone de 0.7 g à 200 g en particulier 0.7 g à 100 g, ou comprenant une quantité d'aleurone de 0.6 g à 180 g.

## Patentansprüche

1. Zusammensetzung, umfassend die gesamte oder einen Teil der Aleuronschicht eines Getreides für ihre Verwendung zur Vorbeugung oder Behandlung der hepatischen oder cerebralen Toxizität, die von mindestens einem Schadstoff induziert wird, der insbesondere in einem Lebensmittel enthalten ist,
wobei der Schadstoff ein PCB oder ein PCB-Gemisch und/oder ein Pestizid ist, ausgewählt aus den Chlororganischen, den Phosphororganischen, den Pyrethroiden, den Carbamaten und den Triazinen,
wobei die verabreichte Aleuronmenge von 0,01 g/kg Körpergewicht (KG) bis 0,5 g/kg KG oder von 0,5 g/kg KG bis 5 g/kg KG liegt,
oder von 0,01 g/kg KG bis 0,05 g/kg KG oder von 0,05 g/kg KG bis 0,1 g/kg KG oder von 0,1 g/kg KG bis 0,5 g/kg KG oder von 0,5 g/kg KG bis 1 g/kg KG oder von 1 g/kg KG bis 3 g/kg KG oder von 3 g/kg KG bis 6 g/kg KG.

2. Zusammensetzung, umfassend die gesamte oder einen Teil der Aleuronschicht eines Getreides für ihre Verwendung nach Anspruch 1, **dadurch gekennzeichnet, dass** der Schadstoff aus einem PCB oder einem PCB-Gemisch ausgewählt ist, das zur Gruppe der "dioxin-like" PCBs gehört, insbesondere ausgewählt aus dem PCB 126 (3,3',4,4',5-Pentachlorbiphenyl), dem PCB 118 (2,3,4,4',5-Pentachlorbiphenyl) und/oder den "nicht dioxin-like" PCBs, insbesondere ausgewählt aus dem PCB 28 (2,4,4'-Trichlorbiphenyl), dem PCB 52 (2,2',5,5'-Tetrachlor-1,1'-Biphenyl), dem PCB 101 (2,2',4,5,5'-Pentachlorbiphenyl), dem PCB 138 (2,2',3,4,4',5'-Hexachlorbiphenyl), dem PCB 153 (2,3',4,4',5-Pentachlorbiphenyl), dem PCB 180 (2,2',3,4,4',5,5'-Heptachlorbiphenyl) oder den Dioxinen oder den Pestiziden.

3. Zusammensetzung, umfassend die gesamte oder einen Teil der Aleuronschicht eines Getreides für ihre Verwendung nach Anspruch 1, **dadurch gekennzeichnet, dass** die Verwendung bei den menschlichen oder tierischen Wesen durchgeführt wird, deren Gewebe PCBs enthalten, insbesondere bei menschlichen Wesen, deren Fettgewebe PCBs in einer Dosis von 14 bis 1700 ng/g enthalten.

4. Zusammensetzung, umfassend die gesamte oder einen Teil der Aleuronschicht eines Getreides für ihre Verwendung nach Anspruch 3, **dadurch gekennzeichnet, dass** die Verwendung bei den menschlichen Wesen durchgeführt wird, deren Serum PCBs in einer Dosis von 0,9 bis 1,5 ng/ml, insbesondere von 5,4 bis 9 ng/ml in besonderen Expositionssituationen und insbesondere von 900 bis 1500 ng/ml bei einer beruflichen Exposition enthält.

5. Zusammensetzung, umfassend die gesamte oder einen Teil der Aleuronschicht eines Getreides für ihre Verwendung nach Anspruch 4, **dadurch gekennzeichnet, dass** die Verwendung bei den menschlichen Wesen durchgeführt wird, deren Muttermilch PCBs in einer Dosis von 238 bis 271 ng/g und insbesondere 1090 ng/g in besonderen Expositionssituationen enthält.

6. Zusammensetzung, umfassend die gesamte oder einen Teil der Aleuronschicht eines Getreides für ihre Verwendung nach einem der Ansprüche 1 oder 2, **dadurch gekennzeichnet, dass** der Schadstoff in Dosen von 3 ng/kg KG/Tag bis 6 mg/kg KG/Tag verabreicht ist,
insbesondere chronisch, insbesondere täglich und vor allem in Dosen von 3 bis 50 ng/kg KG/Tag und insbesondere von 10 bis 40 ng/kg KG/Tag und genauer von 20 bis 30 ng/kg/Tag,
oder insbesondere akut in Dosen über 1 µg/kg PC/Tag, insbesondere von 1 mg/kg KG/Tag bis 3000 mg/kg KG/Tag, genauer von 500 µg/kg KG/Tag bis 6 mg/kg KG/Tag.

7. Zusammensetzung, umfassend die gesamte oder einen Teil der Aleuronschicht eines Getreides für ihre Verwendung nach einem der Ansprüche 1 bis 6, **dadurch gekennzeichnet, dass** das Getreide aus den verschiedenen Weizenvarietäten der Sorte *Triticum* (Weichweizen, Hartweizen, Khorasan-Weizen...), Dinkel, Gerste, Hafer, Hirse, Mais, Reis, Roggen, Buchweizen, Quinoa, Amarant, Sesam, Triticale ausgewählt ist, wobei das Getreide insbesondere Weizen oder ein Gemisch dieser Getreide ist.

8. Zusammensetzung, umfassend die gesamte oder einen Teil der Aleuronschicht eines Getreides für ihre Verwendung nach einem der Ansprüche 1 bis 7, **dadurch gekennzeichnet, dass** die Vorbeugung oder Behandlung beim Menschen oder bei den Tieren, insbesondere den Haustieren oder Nutztieren, durchgeführt wird.

9. Zusammensetzung, umfassend die gesamte oder einen Teil der Aleuronschicht eines Getreides für ihre Verwendung nach einem der Ansprüche 1 bis 8, **dadurch gekennzeichnet, dass** die hepatische und cerebrale Toxizität von einem oxidativen Stress hervorgerufen wird, oder die hepatische und cerebrale Toxizität von einem nicht oxidativen Mechanismus hervorgerufen wird, oder die hepatische und cerebrale Toxizität ausschließlich von einem nicht oxidativen Mechanismus hervorgerufen wird, oder die hepatische und cerebrale Toxizität nicht ausschließlich von einem nicht oxidativen Mechanismus hervorgerufen wird, oder die hepatische und cerebrale Toxizität von einem oxidativen Stress und/oder einem nicht oxidativen Mechanismus hervorgerufen wird.

10. Zusammensetzung, umfassend die gesamte oder einen Teil der Aleuronschicht eines Getreides für ihre Verwendung nach einem der Ansprüche 1 bis 9, **dadurch gekennzeichnet, dass** die hepatische und cerebrale Toxizität ausschließlich von einem nicht oxidativen Mechanismus hervorgerufen wird, wobei die hepatische und cerebrale Toxizität die Erhöhung des Verhältnisses Omega 6 zu Omega 3 im Vergleich zum Verhältnis Omega 6 zu Omega 3 gleich 5 ist.

11. Zusammensetzung, umfassend die gesamte oder einen Teil der Aleuronschicht eines Getreides für ihre Verwendung nach einem der Ansprüche 1 bis 9, **dadurch gekennzeichnet, dass** die hepatische und cerebrale Toxizität von einem oxidativen Stress hervorgerufen wird, wobei die hepatische und cerebrale Toxizität die Erhöhung der lipidischen Peroxydation in der Leber und im Gehirn ist.

12. Zusammensetzung, umfassend die gesamte oder einen Teil der Aleuronschicht eines Getreides für ihre Verwendung nach einem der Ansprüche 1 bis 9, **dadurch gekennzeichnet, dass** die hepatische und cerebrale Toxizität von einem oxidativen Stress und/oder einem nicht oxidativen Mechanismus hervorgerufen wird, wobei die hepatische und cerebrale Toxizität die Verringerung der Aktivität der Citrat-Synthase und/oder die Störung der mitrochondrialen Respirationskette in der Leber und/oder die Störung der Aktivität der Komplexe der mitrochondrialen Respirationskette im Gehirn und/oder die Erhöhung der Aktivität der Transaminasen im Plasma ist.

13. Zusammensetzung, umfassend die gesamte oder einen Teil der Aleuronschicht eines Getreides für ihre Verwendung nach einem der Ansprüche 1 bis 9, **dadurch gekennzeichnet, dass** die Aleuron-Einzeldosis der Zusammensetzung von 0,7 g bis 200 g, vor allem 0,7 g bis 100 g liegt oder von 0,6 g bis 180 g liegt.

14. Emährungs- oder nutrazeutische Zusammensetzung für ihre Verwendung zum Hemmen der hepatischen und cerebralen Toxizität aufgrund von Schadstoffen, wobei die Zusammensetzung eine Aleuronmenge von 0,7 g bis 200 g, vor allem 0,7 g bis 100 g umfasst, oder eine Aleuronmenge von 0,6 g bis 180 g umfasst.

## Claims

1. Composition comprising all or part of the aleurone layer of a cereal for use thereof in the prevention or treatment of liver and brain toxicity induced by at least one pollutant particularly contained in a food,
said pollutant being a PCB or mixture of PCBs and/or a pesticide selected from among organochlorines, organophosphorus compounds, pyrethroids, carbamates and triazines,
the amount of aleurone administered being from 0.01 g/kg bodyweight (BW) to 0.5 g/kg BW or from 0.5 g/kg BW to 5 g/kg BW,
or from 0.01 g/kg BW to 0.05 g/kg BW, or from 0.05 g/kg BW to 0.1 g/kg BW, or from 0.1 g/kg BW to 0.5 g/kg BW, or from 0.5 g/kg BW to 1 g/kg BW, or from 1 g/kg BW to 3 g/kg BW, or from 3 g/kg BW to 6 g/kg BW.

2. Composition comprising all or part of the aleurone layer of a cereal for use thereof according to claim 1, **characterized in that** said pollutant is selected from among a PCB or mixture of PCBs belonging to the "dioxin-like" group of PCBs, in particular selected from among PCB 126 (3,3',4,4',5-pentachlorobiphenyl), PCB 118 (2,3,4,4',5-pentachlorobiphenyl), and/or "non-dioxin-like" PCBs selected in particular from among PCB 28 (2,4,4'-Trichlorobiphenyl), PCB 52 (2,2',5,5'-tetrachloro-1,1'-Biphenyl), PCB 101 (2,2',4,5,5'-Pentachlorobiphenyl), PCB 138 (2,2',3,4,4',5'-hexachlorobiphenyl), PCB 153 (2,3',4,4',5-Pentachlorobiphenyl), PCB 180 (2,2',3,4,4',5,5'-heptachlorobiphenyl, or dioxins or pesticides.

3. Composition comprising all or part of the aleurone layer of a cereal for use thereof according to claim 1, **characterized in that** said use is applied in human or animal subjects having tissues containing PCBs, in particular in human subjects having adipose tissue containing PCBs at a dose ranging from 14 to 1700 ng/g.

4. Composition comprising all or part of the aleurone layer of a cereal for use thereof according to claim 3, **characterized in that** said use is applied in human subjects having serum containing PCBs at a dose ranging from 0.9 to 1.5 ng/ml, in particular from 5.4 to 9 ng/ml in situations of particular exposure, and notably from 900 to 1500 ng/ml for occupational exposure.

5. Composition comprising all or part of the aleurone layer of a cereal for use thereof according to claim 4, **characterized in that** said use is applied in human subjects having breast milk containing PCBs at a dose ranging from 238 to 271 ng/g and in particular 1090 ng/g in situations of particular exposure.

6. Composition comprising all or part of the aleurone layer of a cereal for use thereof according to one of claims 1 or 2, **characterized in that** the pollutant is administered at doses ranging from 3 ng/kg BW/day to 6 mg/kg BW/day,
in particular in chronic manner, in particular daily and particularly at doses ranging from 3 to 50 ng/kg BW/day, in particular 10 to 40 ng/kg BW/day, more particularly from 20 to 30 ng/kg/day,
or in acute manner at doses higher than 1 µg/kg BW/day, in particular from 1 mg/kg BW/day to 3000 mg/kg BW/day, more particularly from 500 µg/kg BW/day to 6 mg/kg BW/day.

7. Composition comprising all or part of the aleurone layer of a cereal for use thereof according to one of claims 1 to 6, **characterized in that** said cereal is selected from among different varieties of wheat of genus *Triticum* (soft wheat, hard wheat, Khorasan wheat ...), spelt, barley, oats, millet, maize, rice, rye, buckwheat, quinoa, amaranth, sesame, triticale, said cereal particularly being wheat or a mixture of these cereals.

8. Composition comprising all or part of the aleurone layer of a cereal for use thereof according to one of claims 1 to 7, **characterized in that** said prevention or treatment takes place in man or animals, in particular pet animals or productive livestock.

9. Composition comprising all or part of the aleurone layer of a cereal for use thereof according to one of claims 1 to 8, **characterized in that** liver and brain toxicity is caused by oxidative stress, or liver and brain toxicity is caused by a non-oxidative mechanism, or liver and brain toxicity is caused solely by a non-oxidative mechanism, or liver and brain toxicity is not solely caused by a non-oxidative mechanism, or liver and brain toxicity is caused by oxidative stress and/or a non-oxidative mechanism.

10. Composition comprising all or part of the aleurone layer of a cereal for use thereof according to one of claims 1 to 9, **characterized in that** liver and brain toxicity is solely caused by a non-oxidative mechanism, the liver and brain toxicity being an increase in the omega 6 to omega 3 ratio compared with the omega 6 to omega 3 ratio of 5.

11. Composition comprising all or part of the aleurone layer of a cereal for use thereof according to one of claims 1 to 9, **characterized in that** liver and brain toxicity is caused by oxidative stress, the liver and brain toxicity being an increase in lipid peroxidation in the liver and brain.

12. Composition comprising all or part of the aleurone layer of a cereal for use thereof according to one of claims 1 to 9, **characterized in that** liver and brain toxicity is caused by oxidative stress and/or a non-oxidative mechanism, the liver and brain toxicity being decreased citrate synthase activity, and/or perturbation of the mitochondrial respiratory chain in the liver, and/or perturbation of the activity of the mitochondrial respiratory chain complexes in the brain, and/or an increase in plasma-transaminase activity.

13. Composition comprising all or part of the aleurone layer of a cereal for use thereof according to one of claims 1 to 9, **characterized in that** the unit dose of aleurone in said composition is from 0.7 g to 200 g, in particular 0.7 g to 100 g or from 0.6 g to 180 g.

14. Food or nutraceutical composition for use thereof to inhibit liver and brain toxicity due to pollutants, said composition comprising an amount of aleurone of 0.7 g to 200 g, in particular 0.7 g to 100 g, or comprising an amount of aleurone of 0.6 g to 180 g.
